# EUROPEAN PATENT SPECIFICATION

(11) **EP 3 668 428 B1**
(45) Date of publication and mention of the grant of the patent: **16.08.2023**
(21) Application number: 18928987.9
(22) Date of filing: 15.08.2018
(51) Int. Cl.: A61M 37/00, A61N 2/00

(54) **MAGNETIC DEVICES FOR ALTERING LOADING ACROSS JOINTS**
MAGNETISCHE VORRICHTUNGEN ZUR VERÄNDERUNG DER BELASTUNG VON GELENKEN
DISPOSITIFS MAGNÉTIQUES POUR MODIFIER LA CHARGE À TRAVERS DES ARTICULATIONS

(30) Priority: 15.08.2017 US 201762545572 P; 15.08.2017 US 201715677822; 08.12.2017 US 201715835802; 11.06.2018 US 201816005641
(43) Date of publication of application: 24.06.2020
(73) Proprietor: Fellowship of Orthopaedic Researchers, Inc., Metairie, LA 70005 (US); Gomboc, LLC, Metairie LA 70005 (US)
(72) Inventor: COOK, Stephen, D., Metairie, LA 70005 (US); SALKED, Samantha, L., Metairie, LA 70005 (US); PATRON, Laura, P., Metairie, LA 70005 (US); NOLAN, Liam, P., Metairie, LA 70005 (US); HARRISON, Michael, C., Metairie, LA 70005 (US)
(74) Representative: Goddar, Heinz J.
(86) International application number: PCT/US2018/046790
(87) International publication number: WO 2020/050811

(56) References cited:
- WO-A1-2017/066774
- WO-A2-2014/015061
- US-A1- 2004 059 423
- US-A1- 2006 271 107
- US-A1- 2007 150 910
- US-A1- 2007 276 378
- US-A1- 2008 306 324
- US-A1- 2008 306 324
- US-B2- 6 599 321
- US-B2- 6 599 321

## Description

### BACKGROUND OF THE INVENTION

Cartilage and other connective tissue in cartilaginous joints can break, fracture, or otherwise become damaged due to injury, age, heredity, or combinations thereof, resulting in pain and/or loss of motion. Often, treatments for such conditions are ineffective in alleviating the pain, or require a long recovery.

For example, hyaline cartilage that covers the surface of bones forming a load-bearing joint allows pain free motion at the joint and absorbs loads placed across the joint during activities of daily living. But these cartilage surfaces can experience wear and damage due to trauma or osteoarthritis, or other factors such as obesity and poor j oint surface alignment, and the exposed bone surfaces can cause significant pain in the joint upon motion. Moreover, the loss of the cartilage surface can diminish its shock absorption capability, which can result in excessive loads being placed across the joint and lead to further damage to the cartilage surfaces and increasing patient symptoms. Reducing load across the j oint is an effective way clinically of reducing the level of pain in the patient, and can be accomplished by several methods, including weight loss, surgery to realign the anatomy, and, in cases of severe cartilage loss or wear, replacement of the joint. However, weight loss may be difficult or slow to achieve, and it may take time before it results in a reduction in load or alleviation of pain. Further, surgery or joint replacement does not leave the joint intact and requires a long recovery time.

As another example, cartilage and components of the intervertebral disc between vertebrae can degenerate, resulting in pain and pressure on the spinal cord. Traction methods, such as manual cervical traction that includes inflatable neck collars and over-the-door traction devices, are meant to relax the soft tissue and separate spinal vertebral segments, therefore creating negative intra-disc pressure, retracting bulging discs, and removing impingements on nerves. However, while methods and devices for supplying traction forces are generally known, they do not provide consistent, continuous low magnitude traction forces in order to maintain disc height and health and to relieve acute and chronic cervical neck pain. In addition, current apparatuses and methods for fixation or fusion of bones and joints to promote healing, relieve pain, and/or reduce future injury of other cartilaginous joints are often insufficient to provide proper stability or otherwise aid in healing or treating the bones and connective tissues involved.

US 2008/306324 A1 discloses a bone screw according to the preamble of claim 1.

Thus, there remains a need in the art for an intervention that effectively reduces loading in cartilaginous joints and that does not substantially alter the anatomy of the joint, thereby requiring a more effective treatment and shorter time to recover.

### SUMMARY OF INVENTION

The present invention relates to a bone screw according to claim 1 and an apparatus according to claim 8 for altering loading across joints or fracture sites.

The scope of the invention is only defined by the appended claims. The examples and embodiments not falling within the scope of the claims as well as all methods of the present description are shown for the purpose of understanding the invention. The methods do not fall within the scope of the present invention.

In embodiments of the bone screw, the lower section of the shaft is attached to the middle section. In some embodiments, the lower section is hermetically sealed to the middle section. In certain embodiments, the lower section is laser welded to the middle section.

In embodiments of the bone screw, the magnet is adhered to at least a portion of the inner wall surface. In some embodiments, the magnet is adhered to at least a portion of the inner wall surface using surgical adhesive. In certain embodiments, the surgical adhesive comprises a medical grade epoxy. In some embodiments of the bone screw, the magnet is sealed to the inner wall surface using laser welding techniques.

In embodiments of the bone screw, the upper section of the shaft comprises an end surface that is configured to receive a driver. In some embodiments, the end surface of the upper section contains a mark that identifies the polarity of the magnet.

In embodiments of the bone screw, the lower section comprises a general conical shape.

In embodiments of the bone screw, the shaft comprises a metal alloy. In certain embodiments, the metal alloy comprises titanium, cobalt chromium, stainless steel, or a combination thereof.

In certain embodiments of the bone screw, the shaft comprises a length of about 5 to about 100 mm. In some embodiments, the length is about 10 to about 80 mm. In other embodiments, the length is about 16 to about 25 mm.

In embodiments of the invention, the shaft comprises a diameter of about 2 to about 12 mm. In some embodiments, the diameter is about 3 to about 10 mm. In other embodiments, the diameter is about 5 to about 6 mm.

In embodiments of the apparatus, the first surface comprises a contouring profile configured to the contouring profile of at least a portion of a vertebra, such as the vertebral body of the vertebra. In some embodiments, the first surface comprises a contouring profile configured to the contouring profile of at least a portion of the vertebral body of two or more adjacent vertebrae. In certain embodiments, the vertebrae are cervical vertebrae. In other embodiments, the vertebrae are lumbar or thoracic vertebrae.

In embodiments of the apparatus, the at least one aperture of the first end section comprises a diameter of about 2 mm to about 12 mm.

In embodiments of the apparatus, the at least one aperture of the second end section comprises a length of about 2.5 mm to about 15 mm. In some embodiments, the at least one aperture of the second end section comprises a width of about 2 mm to about 12 mm.

In embodiments of the invention, the width of the first end section and the second end section is greater than the width of the middle section.

In embodiments of the invention, two apertures are in the first end section and two apertures are in the second end section.

In embodiments of the invention, the bone plate further comprises one or more apertures for receiving a locking tab. In some embodiments, the one or more apertures for receiving a locking tab are in the middle section of the plate.

In embodiments of the invention, the plate comprises a metal alloy, polymer, a composite of polymers and fibers, or a combination thereof. In some embodiments, the metal alloy comprises titanium, cobalt chromium, stainless steel, or a combination thereof. In certain embodiments, the polymer comprises polyetheretherketone, polyurethane, or a combination thereof. In further embodiments, the composite comprises carbon fiber reinforced polyetheretherketone.

In embodiments, the cartilaginous joint is a knee joint and the method comprises implanting one or more magnetic devices in the distal femur and one or more magnetic devices in the proximal tibia, wherein the one or more magnetic devices in the femur and the one or more magnetic devices in the tibia are oriented to generate a repulsive magnetic force therebetween. In some embodiments, the one or more magnetic devices in the femur are implanted in a condyle of the femur. In certain embodiments, the one or more magnetic devices in the femur are implanted in the medial condyle of the femur, such as implanted into the medial surface of the medial condyle. In other embodiments, the one or more magnetic devices in the femur are implanted in the lateral condyle of the femur, such as implanted into the lateral surface of the lateral condyle. In certain embodiments, the one or more magnetic devices in the femur are implanted in both the medial condyle and the lateral condyle of the femur.

In some embodiments, the one or more magnetic devices in the tibia are implanted in a condyle of the tibia. In certain embodiments, the one or more magnetics devices in the tibia are implanted in the medial condyle of the tibia, such as implanted into the medial surface of the medial condyle. In other embodiments, the one or more magnetic devices in the tibia are implanted in the lateral condyle of the tibia, such as implanted into the lateral surface of the lateral condyle. In certain embodiments, the one or more magnetic devices in the tibia are implanted in both the medial condyle and the lateral condyle of the tibia.

In some embodiments, the one or more magnetic devices in the femur are implanted in the medial condyle of the femur, and the one or more magnetic devices in the tibia are implanted in the medial condyle of the tibia. In certain embodiments, the one or more magnetic devices in the femur are implanted in the lateral condyle of the femur, and the one or more magnetic devices in the tibia are implanted in the lateral condyle of the tibia.

In some embodiments, the number of magnetic devices implanted in the femur ranges from one to five, i.e., one, two, three, four, or five magnetic devices. In some embodiments, the number of magnetic devices implanted in the tibia ranges from one to five, i.e., one, two, three, four, or five magnetic devices. In certain embodiments, the number of magnetic devices implanted in the femur is the same as the number of magnetic devices implanted in the tibia. In other embodiments, the number of magnetic devices implanted in the femur is different than the number of magnetic devices implanted in the tibia.

In embodiments, the magnetic devices are implanted in a configuration such that the one or more magnetic devices of the femur are maintained at a prescribed distance, or a range of distances, from the one or more magnetic devices of the tibia while the knee undergoes flexion and/or extension. In some embodiments, the magnetic devices are implanted in a curved configuration generally along an anatomic curve of the condyle of the femur and/or tibia. For instance, the magnetic devices are implanted into the medial surface of the medial condyle of the femur and/or tibia in a configuration generally along the anterior-posterior curve of the surface of the medial condyle of the femur and/or tibia. Alternatively or in addition, the magnetic devices are implanted into the lateral surface of the lateral condyle of the femur and/or tibia in a configuration generally along the anterior-posterior curve of the surface of the lateral condyle of the femur and/or tibia. In other embodiments, the magnetic devices are implanted in a linear configuration.

In embodiments, the cartilaginous joint is an acetabulofemoral joint and the method comprises implanting one or more magnetic devices in the proximal femur and one or more magnetic devices in the hip bone adjacent to the acetabulum, wherein the one or more magnetic devices in the femur and the one or more magnetic devices in the hip bone are oriented to generate a repulsive magnetic force therebetween. In some embodiments, the one or more magnetic devices in the femur are implanted in the femoral head.

In embodiments, the cartilaginous joint is an ankle joint and the method comprises implanting one or more magnetic devices in the distal tibia and/or fibula and one or more magnetic devices in the talus, wherein the one or more magnetic devices in the tibia and/or fibula and the one or more magnetic devices in the talus are oriented to generate a repulsive magnetic force therebetween. In some embodiments, one or more magnetic devices are implanted in both the tibia and the fibula. In other embodiments, one or more magnetic devices are implanted in the tibia only. In certain embodiments, one or more magnetic devices are implanted in the proximal talus. In some embodiments, the magnetic device(s) are implanted into the medial surface of the tibia and/or fibula and into the medial surface of the talus. In other embodiments, the magnetic device(s) are implanted into the lateral surface of the tibia and/or fibula and into the lateral surface of the talus. In yet other embodiments, the magnetic device(s) are implanted into both the medial surface and lateral surface of the tibia and/or fibula and into both the medial surface and lateral surface of the talus.

In embodiments, the cartilaginous joint is an intervertebral joint and the method comprises implanting one or more magnetic devices in a first vertebra that is superior to the intervertebral joint and implanting one or more magnetic devices in a second vertebra that is inferior to the intervertebral joint, wherein the one or more magnetic devices in the first vertebra and the one or more magnetic devices in the second vertebra are oriented to generate a repulsive magnetic force therebetween. In some embodiments, the one or more magnetic devices are implanted in the vertebral body of the first and the second vertebra.

An aspect not belonging to the invention relates to (i) methods of providing cervical traction to reduce pain in an intervertebral joint of a first vertebra superior to the joint and a second vertebra inferior to the joint, (ii) methods of treating pain caused by a herniated disk between a first vertebra that is superior to the herniated disk and a second vertebra that is inferior to the herniated disk, and (iii) methods of treating a herniated disk between a first vertebra that is superior to the herniated disk and a second vertebra that is inferior to the herniated disk. In embodiments, the method comprises implanting one or more magnetic devices, such as at least two magnetic devices, in the first vertebra, and implanting one or more magnetic devices, such as at least two magnetic devices, in the second vertebra. The one or more magnetic devices in the first vertebra and the one or more magnetic devices in the second vertebra are oriented to generate a repulsive magnetic force therebetween.

In alternative embodiments, the methods not belonging to the present invention comprise affixing one or more magnetic devices onto the surface of the bones that form the cartilaginous joint. For instance, one or more magnetic devices is affixed to the surface of the distal femur and one or more magnetic devices onto the surface of the proximal tibia, wherein the one or more magnetic devices on the surface of the distal femur and the one or more magnetic devices on the surface of the proximal tibia are oriented to generate a repulsive magnetic force therebetween. The magnetic device(s) is affixed to the medial surface, the lateral surface, or both the medial and lateral surfaces, of the distal femur and the proximal tibia.

An aspect not belonging to the invention relates to a method of stabilizing a fused intervertebral joint between a first vertebra and a second vertebra. The method comprises fastening a bone plate as described above via two or more bone screws as described above to the first vertebra and the second vertebra, wherein the plate bridges the fused intervertebral joint and at least one bone screw is inserted into each of the first vertebra and the second vertebra.

In some embodiments, the bone screws are inserted through the apertures of the plate to fasten the plate to the first vertebra and to the second vertebra, in which the bone screw(s) inserted through the one or more apertures of the first end section fastens the first end section to the first vertebra, and the bone screw(s) inserted though the one or more apertures of the second end section fastens the second end to the second vertebra. In certain embodiments, the bone screws are oriented to generate an attractive magnetic force between the magnet(s) of the bone screw(s) inserted through the one or more apertures of the first end section and the magnet(s) of the bone screw(s) inserted though the one or more apertures of the second end section.

An aspect not belonging to the invention relates to a method of stabilizing fused intervertebral joints between three or more vertebrae. The method comprises fastening a bone plate as described above via three or more bone screws as described above to each of the three or more vertebrae, wherein the plate bridges each fused intervertebral joint between the three or more vertebrae, and at least one bone screw is inserted into each of vertebrae.

An aspect not belonging to the invention relates to a method of preventing or reducing deterioration of a nonfused intervertebral joint that is superior or inferior to a fused intervertebral joint. The method comprises inserting one or more bone screws into a first vertebra and one or more bone screws into a second vertebra that form the nonfused intervertebral joint. The first vertebra also forms a fused intervertebral joint with an adjacent vertebra. In certain embodiments, the bone screws are oriented generate a repulsive magnetic force between the magnet of one or more bone screws inserted into the first vertebra and the magnet of one or more bone screws inserted into the second vertebra.

An aspect not belonging to the present invention relates to methods of promoting healing across a fracture site. These methods comprise implanting one or more magnetic devices in the bone on each side of the fracture site, in which the magnetic devices are oriented to generate an attractive magnetic force between the magnetic devices on each of the fracture site.

### BRIEF DESCRIPTION OF THE DRAWING FIGURES

The present disclosure will be further explained with reference to the attached drawing figures, wherein like structures are referred to by like numerals throughout the several views. The drawing figures shown are not necessarily to scale, with emphasis instead generally being placed upon illustrating the principles of the present disclosure, and some features may be exaggerated to show details of particular components. In addition, any measurements, specifications, and the like shown in the drawing figures, or described below, are intended to be illustrative, and not restrictive. Therefore, specific structural and functional details disclosed herein are not to be interpreted as limiting, but merely as a representative basis for teaching one skilled in the art to variously employ the devices and methods of their use.
Figure 1 is a side view of a cylindrical magnetic device according to embodiments of the present invention.
Figure 2 is an exploded view of a cylindrical magnetic device according to embodiments of the present invention.
Figure 3 is a perspective view of a rectangular prism magnetic device according to embodiments of the present invention.
Figure 4 is an exploded view of a rectangular prism magnetic device according to embodiments of the present invention.
Figure 5 is a side view of a bone screw according to embodiments of the present invention.
Figure 6 is an exploded view of a bone screw according to embodiments of the present invention.
Figure 7 is a cross-sectional side view of the shaft of a bone screw according to embodiments of the present invention.
Figures 8A and 8B are different views of a bone screw according to embodiments of the present invention. Figure 8A is a top view of a bone screw, and Figure 8B is a cross-sectional bottom view of a bone screw.
Figure 9 is a side view of a disc-shaped housing magnetic device according to embodiments of the present invention.
Figure 10 is an exploded view of a disc-shaped housing magnetic device according to embodiments of the present invention.
Figures 11A, 11B, and 11C are different views of a magnetic device having the shape of a plate attached to a casing that encloses a magnet, according to embodiments of the present invention. Figure 11A is a top view of magnetic device, Figure 11B is a side view of the magnetic device, and Figure 11C is a perspective view of the magnetic device.
Figure 12 is a top view of a bone plate according to embodiments of the present invention.
Figures 13A, 13B, and 13C are different views of a bone plate according to embodiments of the present invention. Figure 13A is a side view of a bone plate, Figure 13B is a cross-sectional end view of a bone plate, and Figure 13C is a cross-sectional end view of a bone plate.
Figure 14 is a top view of a bone plate according to embodiments of the present invention.
Figure 15 is a top view of a bone plate according to embodiments of the present invention.
Figures 16A and 16B are different views of a bone plate according to embodiments of the present invention. Figure 16A is a side view of a bone plate, and Figure 16B is a cross-sectional side view of a bone plate.
Figures 17A and 17B are different views of a bone plate with bone screws according to embodiments of the present invention. Figure 17A is a top view of a bone plate with bone screws, and Figure 17B is a perspective view of a bone plate with bone screws.
Figure 18 is a cross-sectional side view of a bone plate with bone screws according to embodiments of the present invention.
Figures 19A and 19B are different views of a bone plate with bone screws according to embodiments of the present invention. Figure 19A is a top view of a bone plate with bone screws, and Figure 19B is a side view of a bone plate with bone screws.
Figures 20A and 20B are different views of a locking tab according to embodiments of the present invention. Figure 20A is a top view of a locking tab, and Figure 20B is a side view of a locking tab.
Figures 21A and 21B are different views of a knee joint at 0° flexion with cylindrical magnetic devices implanted into the medial surface of the medial condyle of the femur and tibia, according to embodiments of the present invention. Figure 21A is a medial view of the knee joint, and Figure 21B is an anterior view of the knee joint.
Figures 22A and 22B are different views of a knee joint at 0° flexion with cylindrical magnetic devices implanted into the medial surface of the medial condyle of the femur and tibia, according to embodiments of the present invention. Figure 22A is a medial view of the knee joint, and Figure 22B is an anterior view of the knee joint.
Figures 23A and 23B are different views of a knee joint at 0° flexion with bone-screw magnetic devices implanted into the medial surface of the medial condyle of the femur and tibia, according to embodiments of the present invention. Figure 23A is a medial view of the knee joint, and Figure 23B is an anterior view of the knee joint.
Figures 24A and 24B are different views of a knee joint at 0° flexion with cylindrical magnetic devices implanted into the medial surface of the medial condyle of the femur and tibia, according to embodiments of the present invention. Figure 24A is a medial view of the knee joint, and Figure 24B is an anterior view of the knee joint.
Figures 25A and 25B are different views of a knee joint at 0° flexion with rectangular prism-shaped magnetic devices implanted into the medial surface of the medial condyle of the femur and tibia, according to embodiments of the present invention. Figure 25A is a medial view of the knee joint, and Figure 25B is an anterior view of the knee joint.
Figures 26A and 26B are different views of a knee joint at 0° flexion with a cylindrical magnetic device implanted into the medial surface of the medial condyle of the femur and a rectangular prism-shaped magnetic device implanted into the medial surface of the medial condyle of the tibia, according to embodiments of the present invention. Figure 26A is a medial view of the knee joint, and Figure 26B is an anterior view of the knee joint.
Figures 27A and 27B are different views of a knee joint at 0° flexion with a cylindrical magnetic device implanted into the medial surface of the medial condyle of the femur and a disc-shaped magnetic device implanted into the medial surface of the medial condyle of the tibia, according to embodiments of the present invention. Figure 27A is a medial view of the knee joint, and Figure 27B is an anterior view of the knee joint.
Figures 28A, 28B, and 28C are medial views of a knee joint at different angles of flexion, in which cylindrical magnetic devices are implanted into the medial surface of the medial condyle of the femur and the tibia, according to embodiments of the present invention. Figure 28A is a medial view of the knee joint at 0° flexion, Figure 28B is a medial view of the knee joint at 45° flexion, and Figure 28C is a medial view of the knee joint at 90° flexion. The arrows in the magnetic devices represent north pole direction.

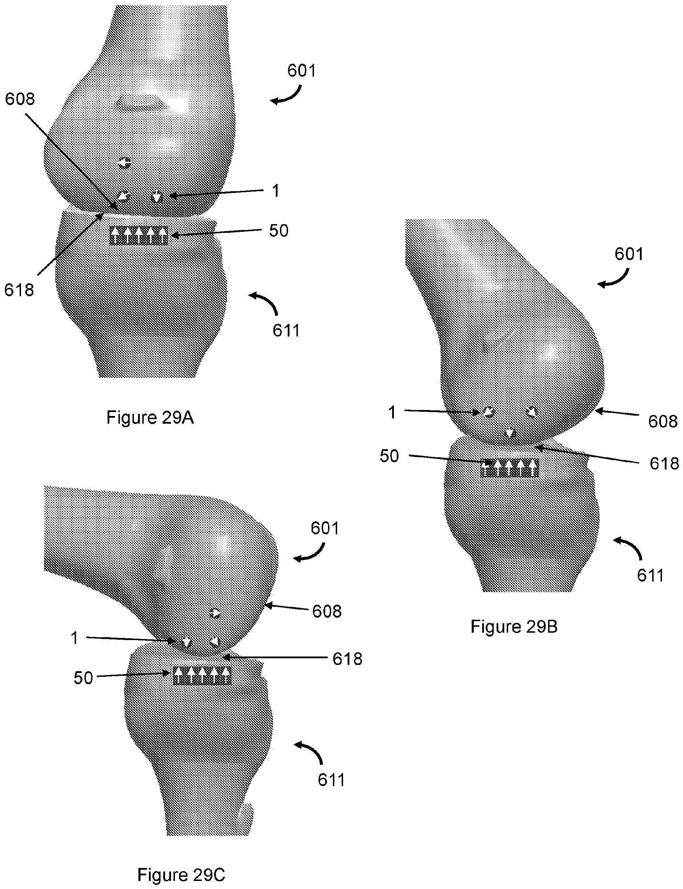

Figures 29A, 29B, and 29C are medial views of a knee joint at different angles of flexion, in which cylindrical magnetic devices are implanted into the medial surface of the medial condyle of the femur and a rectangular prism-shaped magnetic device is implanted into the medial surface of the medial condyle of the tibia, according to embodiments of the present invention. Figure 29A is a medial view of the knee joint at 0° flexion, Figure 29B is a medial view of the knee joint at 45 ° flexion, and Figure 29C is a medial view of the knee joint at 90° flexion. The arrows in the magnetic devices represent north pole direction.
Figures 30A and 30B are different views of a knee joint at 0° flexion with magnetic devices affixed to the medial surface of the medial condyle of the femur and tibia, in which the magnetic devices have the shape of a plate attached to a casing that encloses a magnet, according to embodiments of the present invention. Figure 30A is a medial view of the knee joint, and Figure 30B is an anterior view of the knee joint.
Figures 31A and 31B are different views of an intervertebral joint with bone screw magnetic devices implanted in a vertebra superior to the joint and in a vertebra inferior to the joint, according to embodiments of the present invention. Figure 31A is an anterior view of the intervertebral joint, and Figure 31B is a medial view of the intervertebral joint.
Figures 32A and 32B are different views of an intervertebral joint with disc-shaped magnetic devices implanted in a vertebra superior to the joint and in a vertebra inferior to the joint, according to embodiments of the present invention. Figure 32A is an anterior view of the intervertebral joint, and Figure 32B is a lateral view of the intervertebral joint.
Figures 33A and 33B are different views of a bone plate fastened via bone screws to vertebrae of a fused intervertebral joint according to embodiments of the invention. Figure 33A is a coronal plane view of a bone plate fastened via bone screws to vertebrae of a fused intervertebral joint, and Figure 33B is a sagittal plane view of a bone plate fastened via bone screws to vertebrae of a fused intervertebral joint.
Figures 34A and 34B are different views of a bone plate fastened via bone screws to vertebrae of a fused intervertebral joint according to embodiments of the invention. Figure 34A is a coronal plane view of a bone plate fastened via bone screws to vertebrae of a fused intervertebral joint, and Figure 34B is a sagittal plane view of a bone plate fastened via bone screws to vertebrae of a fused intervertebral joint.
Figure 35 is a top view of a bone plate with bone screws and the direction of the magnetic north poles of the bone screws according to embodiments of the present invention.
Figures 36A and 36B are different views of bone screws inserted into vertebrae of a fused intervertebral joint and additional screws inserted into adjacent vertebrae, according to embodiments of the invention. Figure 36A is a coronal plane view of bone screws inserted into vertebrae of a fused intervertebral joint and additional screws inserted into adjacent vertebrae, and Figure 36B is a sagittal plane view of bone screws inserted into vertebrae of a fused intervertebral joint and additional screws inserted into adjacent vertebrae.
Figure 37 is a coronal plane view of bone screws inserted into vertebrae of a fused intervertebral joint and additional screws inserted into adjacent vertebrae, and the direction of the magnetic north poles of the bone screws according to embodiments of the present invention.
Figures 38A and 38B are different views of a bone plate fastened via bone screws to vertebrae of a fused intervertebral joint and additional bone screws inserted into adjacent vertebrae, according to embodiments of the invention. Figure 38A is a coronal plane view of a bone plate fastened via bone screws to vertebrae of a fused intervertebral joint and additional bone screws inserted into adjacent vertebrae, and Figure 38B is a sagittal plane view of a bone plate fastened via bone screws to vertebrae of a fused intervertebral joint and additional bone screws inserted into adjacent vertebrae.
Figure 39 is a top view of a bone plate with bone screws and additional bone screws, and the direction of the magnetic north poles of the bone screws according to embodiments of the present invention.
Figure 40 is a perspective view of bone plate with bone screws and additional bone screws according to embodiments of the present invention
Figures 41A and 41B are a coronal view (Figure 41A) and a sagittal view (Figure 41B) of bone screws inserted into adjacent vertebrae, according to embodiments of the invention.
Figures 42A and 42B are a coronal view (Figure 42A) and a sagittal view (Figure 42B) of a disc-shaped housing inserted into adjacent vertebrae, according to embodiments of the invention.
Figures 43A and 43B are views of the magnet configuration studied in Example 2.

### DETAILED DESCRIPTION OF THE INVENTION

This invention provides surgically implanted magnetic devices for altering the loading across joints.

Upon insertion into bone, the magnetic devices can be oriented to create repulsive or attractive forces between the devices. In other words, the orientation of the devices direct the polarity of the magnet within the devices to generate repulsive or attractive magnetic forces between the magnets, and hence between the devices. Thus, if a magnetic device is inserted into each of two adjacent bones, or into each of two different portions of the same bone, each device can be oriented such that the polarity of the magnet(s) inside the devices generates a repulsive force that will push the adjacent bones or the portions of the same bone away from each other; or, alternatively, each magnetic device can be oriented such that the polarity of the magnet(s) inside the devices generates an attractive force that will push the adjacent bones or the portions of the same bone away from each other.

In some aspects of the invention, the devices can partially unload a joint to reduce the loading experienced by the bone and tissues at that joint. In cases in which the joint is a cartilaginous joint, the unloading can result in a reduction in stress across the cartilage in the joint, including the cartilage on the bone surfaces. Unloading of the joint may be accomplished by implanting magnetic devices into or onto the bones that form the joint. The anatomy of the internal joint remains intact. The repulsive forces generated by alignment of the magnetic fields of the devices act to reduce the load across the joint, including during activities. Without being bound by theory, the reduction in load across the joint reduces contact stresses (pressures) on the joint surfaces, resulting in less pain in the patient. The reduction of load also prevents excessive forces from causing further damage to the cartilage surfaces and joint. In cases in which the joint is an intervertebral joint, the repulsive forces can cause traction, and therefore can be used as a treatment for a herniated disk and/or reduce pain across the j oint.

In other aspects of the invention, the devices can provide additional loading across a joint or even across a fracture. Loading of the joint may be accomplished by implanting magnetic devices into or onto the bones that form the joint. The anatomy of the internal joint remains intact. Without being bound by theory, the increase in load across a joint can provide stability and/or compression across the joint. If the intervertebral joint is fused/undergoing fusion, the increase in load can help promote the fusion process. In the case of loading across a fracture, magnetic devices may be implanted into or onto the bone(s) on either side of the fracture site, which may provide stability and/or compression that can promote the healing of the fracture.

The magnetic devices of the invention can be used with a plate that can extend across adjacent bones or portions of the same bone. The plate in combination with the devices can be used to stabilize the adjacent bones, or portions of the same bone. As an example, the plate and devices can provide stability and compression to two or more adjacent vertebrae while a surgically-induced fusion process occurs.

### Magnetic Devices

The magnetic devices may comprise a magnet enclosed within a casing. The casing may comprise a titanium alloy or other type of acceptable biomaterial that is known in the art. The magnet may be hermetically sealed within the casing. In some embodiments, the casing may comprise two or more components (e.g., an upper component and a lower component), in which the two or more components may be laser-welded together in order to create a hermetically-sealed environment for the magnet.

The casing comprises an outer surface that, upon implantation, will face the biological environment, and an inner surface that faces the magnet. The outer surface may be smooth or may comprise surface modifications that stabilize and/or prevent movement, such as rotation, of the device once it is implanted. In some embodiments, the surface modifications may adhere the magnetic device to the biological environment or may generate friction between the magnetic device and the biological environment. The surface modifications may comprise a roughened surface or a pattern of protrusions that are raised from the surface. The surface modifications may also comprise screw thread(s) or a grooved design, such as in embodiments in which the casing is a bone screw, or any other acceptable surgical configuration.

The casing may be fabricated with a metal alloy known in the art for orthopaedic applications, for example, titanium, cobalt chromium, or stainless steel. In certain embodiments, the casing or plate may comprise a polymer, such as PEEK or polyurethane, or a combination thereof. In alternative embodiments, the casing or plate may comprise composites of polymers and fibers, such as carbon fiber-reinforced PEEK.

The magnet within the casing may comprise materials known in the art. For example, the magnet may be iron-based, nickel-based, cobalt-based, or an alloy of rare-earth metals. In certain embodiments, the magnet may be a rare-earth magnet, which generally has strong attraction and repulsion forces and has high retentive capacity and resistance to demagnification. In a preferred embodiment, the rare-earth magnet is an alloy of neodymium, iron, and boron ("NdFeB"). NdFeB magnets may provide strong permanent magnetism, high retentive capacity, and resistance to demagnetization.

The magnetic device may be generally any geometric shape, such as a cylinder, disc, prism (including rectangular prism, hexagonal prism, triangular prism, cube, etc.), cone, and pyramid. In some embodiments, the magnetic device may comprise a bone screw, which is described further below.

The shape of the magnetic device may be primarily determined by the shape of the casing. In some embodiments, the magnet of the magnetic device may comprise the same general shape as the casing. For example, if the casing is cylindrical, the magnet within the casing may also be generally cylindrical; if the casing is in the shape of a rectangular prism, the magnet within the casing may also be generally in the shape of a rectangular prism; if the casing is disc-shaped, the magnet within the casing may also be generally disc-shaped. In embodiments in which the casing is in the form of a bone screw, the magnet within the casing may be cylindrical. In embodiments in which the casing is shaped like an arc, such as if the casing were attached to a plate, the magnet within the casing may also be in the shape of an arc.

The magnetic device may comprise a size appropriate for the bone in which it is being implanted and for generating the desired reduction in force across the joint. For example, cylindrical magnetic devices may have a diameter of about 2 mm to about 20 mm, or about 3 mm to about 15 mm, or about 4 mm to about 10 mm; and a length of about 3 mm to about 100 mm, or about 5 mm to about 40 mm, or about 10 mm to about 30 mm, or about 15 mm to about 25 mm. The size of the cylindrical magnetic device may also depend on the bone in which the device is being implanted. For example, in some embodiments, cylindrical magnetic devices may comprise: (i) for implantation in a femur, a diameter of about 4 mm to about 15 mm and a length of about 10 mm to about 30 mm; (ii) for implantation in a tibia, a diameter of about 3 mm to about 10 mm and a length of about 15 mm to about 25 mm; (iii) for implantation in cervical vertebrae, a diameter of about 3.5 mm to about 6 mm and a length of about 8 mm to about 100 mm; and (iv) for implantation in lumbar vertebrae, a diameter of about 4 mm to about 10 mm and a length of about 15 mm to about 40 mm.

As another example, rectangular prism-shaped magnetic devices may comprise a length, width, and height of about 5 mm to about 40 mm, or about 12 mm to about 30 mm. Disc-shaped magnetic devices may comprise a circular diameter of about 8 mm to about 40 mm, or about 12 mm to about 30 mm, and a height of about 2 mm to about 15 mm, or about 3 mm to about 10 mm.

The magnet may be magnetized in a radial, axial, or off-axial direction. The magnetic field generated by the magnets may be of any geometric shape, including disc-shaped, circular, rectangular, oval, ellipsoid (with an axial magnetic orientation), etc. In certain embodiments, cylindrical magnetic devices are magnetized in a radial direction. In some embodiments, disc-shaped magnetic devices are magnetized in an axial direction.

The direction of the pole magnetization of the magnet inside of the casing may be indicated by a mark on the outer surface of the casing. The mark may be a different color or shade than the color of rest of the outer surface of the casing, such as a dark-colored mark; in such a case, the mark may be created, for instance, using laser-etching. The mark may also be a physical feature on the outer surface of the casing, such as a notch or a raised groove.

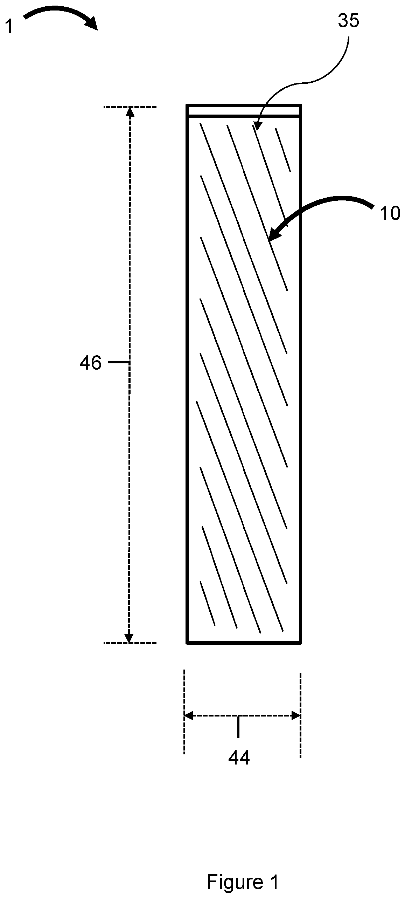

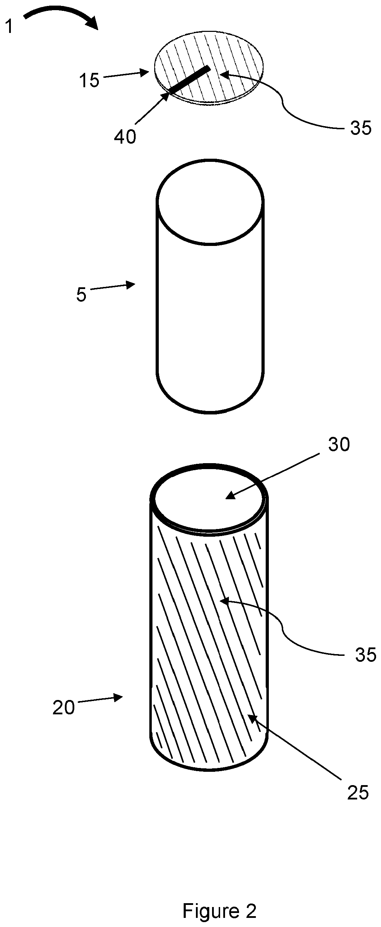

Figures 1, 2, 3, and 4 show examples of a magnetic devices according to embodiments of the invention. Figures 1 and 2 show a cylindrical magnetic device **1** comprising a magnet **5** enclosed within a casing **10**. The casing **10** comprises an upper component **15** and a lower component **20** that are laser-welded together in order to create a hermetically-sealed environment for the magnet **5**. The casing **10** comprises an outer surface **25** that, upon implantation, faces the biological environment, and an inner surface **30** that faces the magnet **5**. The outer surface **25** comprises surface modifications **35** that stabilize and/or prevent movement of the device **1** once it is implanted. The outer surface **25** also comprises a mark **40** to show the direction of the pole magnetization of the magnet **5**. In addition, the cylindrical magnetic device comprises a diameter **44** and a length **46**.

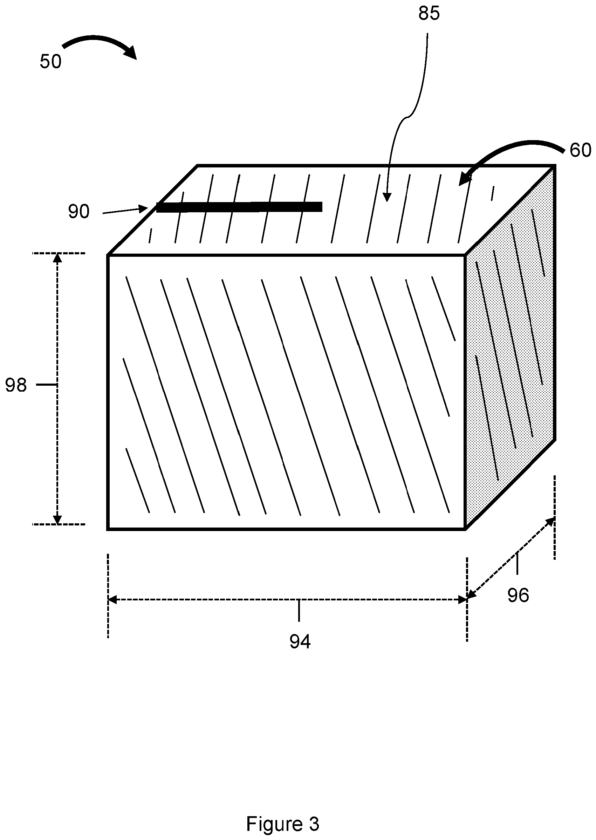

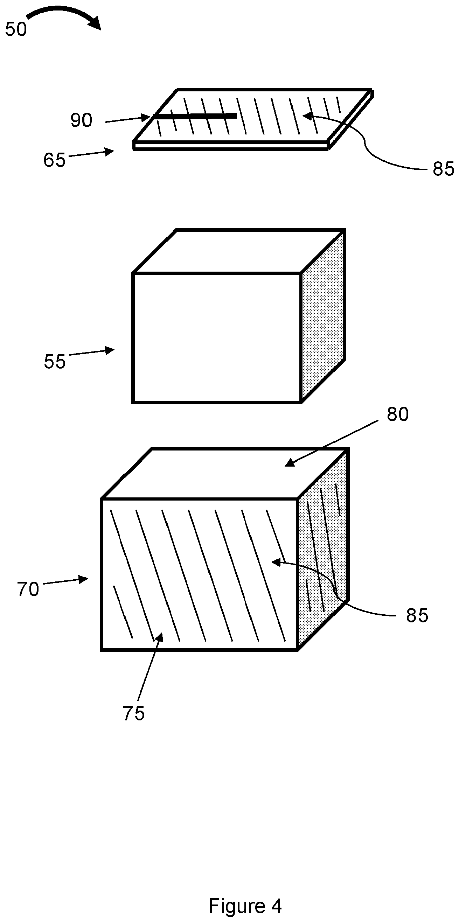

Figures 3 and 4 show a rectangular prism-shaped magnetic devices **50** comprising a magnet **55** enclosed within a casing **60**. The casing **60** comprises an upper component **65** and a lower component **70** that are laser-welded together in order to create a hermetically-sealed environment for the magnet **55**. The casing **60** comprises an outer surface **75** that, upon implantation, faces the biological environment, and an inner surface **80** that faces the magnet **55**. The outer surface **75** comprises surface modifications **85** that stabilize and/or prevent movement of the device **50** once it is implanted. The outer surface **75** also comprises a mark **90** to show the direction of the pole magnetization of the magnet **55**. In addition, the cylindrical magnetic device comprises a length **94**, a width **96**, and a height **98**.

The magnet may be magnetized in a radial, axial, or off-axial direction. The magnetic field generated by the magnets may be of any geometric shape, including disc-shaped, circular, rectangular, oval, ellipsoid (with an axial magnetic orientation), etc. In certain embodiments, cylindrical magnetic devices are magnetized in a radial direction. In some embodiments, disc-shaped magnetic devices are magnetized in an axial direction.

The direction of the pole magnetization of the magnet inside of the casing may be indicated by a mark on the outer surface of the casing. The mark may be a different color or shade than the color of rest of the outer surface of the casing, such as a dark-colored mark; in such a case, the mark may be created, for instance, using laser-etching. The mark may also be a physical feature on the outer surface of the casing, such as a notch or a raised groove.

### Bone Screws

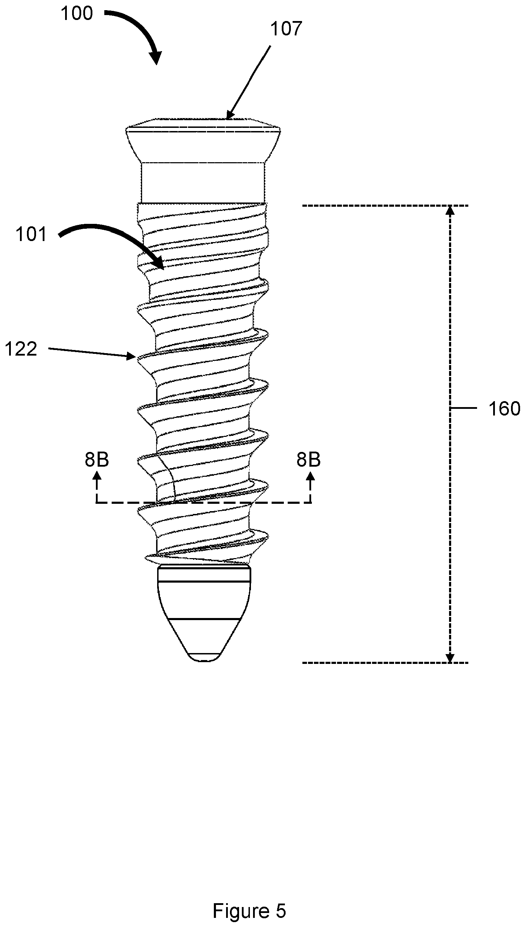

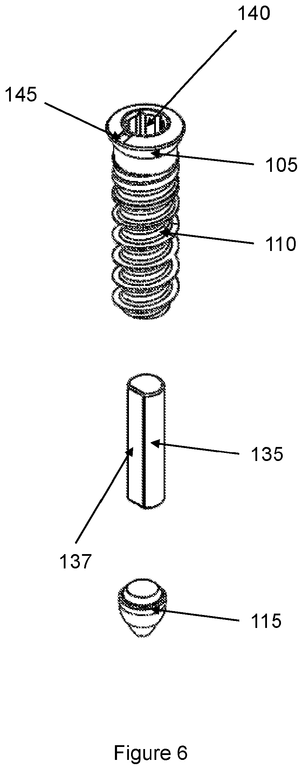

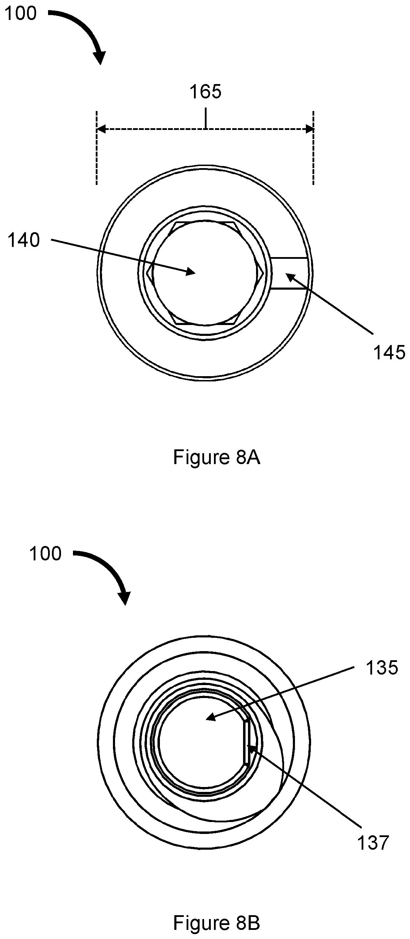

In some embodiments, the magnetic device may be a bone screw. With reference to Figures 5, 6, 7, 8A, and 8B, in certain embodiments, bone screw **100** may comprise a casing **101** that comprises a shaft **103** having an upper section **105**, a middle section **110**, and a lower section **115** (which together form the casing as described above). The shaft **103** may comprise a cross-section that is generally circular.

The shaft **103** may comprise an outer wall **120**, onto which there are a plurality of threads **122**. The threads **122** may have a pitch, depth, and shape that are known in the art for threads of orthopaedic screws, including cortical and cancellous screws. For example, the threads may have any shape as known in the art for drilling into bone, including but not limited to V-thread, buttress thread, reverse buttress, and square thread.

The upper section **105** of the shaft **103** may also be considered as the head of the screw **100**. The top surface **107** of the upper section **105** may comprise a drive **140** that is configured for insertion of a driver, such as 2.5 mm tapered hex driver. The drive **140** may also be configured for insertion of other types of drivers, for instance, Philips-head drivers or flat-head drivers.

The shaft **103** may comprise an inner wall surface **125** that is a result of, or is defined by, a bore **130**. The bore **130** may be located generally throughout the entire middle section **110** of the shaft **103**. A magnet **135** may be housed in the bore **130**, and may be entirely encased within the shaft **103**, including by the outer wall **120** and by the lower section **115**. In certain embodiments, the bore **130** may be generally cylindrical in shape, and therefore the magnet **135** in the bore **130** also may be generally cylindrical in shape. Other shapes are also envisioned for the magnet. For example, the magnet may be ring-shaped, i.e., the magnet itself may comprise a bore through its center along the long axis of the magnet.

The magnet **135** may be prevented from rotating inside the bore **130**. In some embodiments, the bore **130** may be generally cylindrical in shape but with at least one flattened side **137**, and consequently the magnet **135** in the bore **130** also may be generally cylindrical in shape, but with a flattened side **137**. In other embodiments, the bore **130** may be generally cylindrical in shape but may have more than one flattened side, and the magnet **135** in the bore **130** may also be generally cylindrical in shape but may have more than one flattened side (not shown). Alternatively, the magnet **135** may be prevented from rotating inside the bore **130** by gluing or laser welding the magnet **135** to one or more portions of the inner wall surface **125** of the shaft **103**. For example, the magnet may be glued in place with a surgical adhesive such as medical grade epoxy. As yet another alternative, the magnet **135** may be tapered to generate a press fit within the bore **130** of the shaft **103** (not shown).

The magnet **135** may be magnetized in the radial or axial direction. In some embodiments, the orientation, i.e., the direction of the pole magnetization, of the magnet **135** inside of the bore **130** may be indicated by a mark **145** on the top surface **107** of the upper section **105** of the screw **100**. The mark **145** may be a different color or shade than the color of rest of the upper section **105** of the screw **100**, e.g., a dark-colored mark; in such a case, the mark **145** may be created, for instance, using laser-etching. The mark **145** may also be a physical feature on the top surface **107** of the upper section **105** of the screw **100**, such as a notch or a raised groove.

The shaft **103** may be fabricated with a metal alloy known in the art for orthopaedic applications, for example, titanium, cobalt chromium, or stainless steel. In certain embodiments, the upper section **105** and the middle section **110** of the shaft **103** may be fabricated as one continuous component **117**. In some embodiments, the lower section **115** may be fabricated as a separate component that is attached to the continuous component **117**. The attachment of the lower section **115** to the continuous component **117** may create a hermetically sealed environment within the bore **130** of the shaft. In certain embodiments, the lower section **115** is laser-welded to the continuous component **117**.

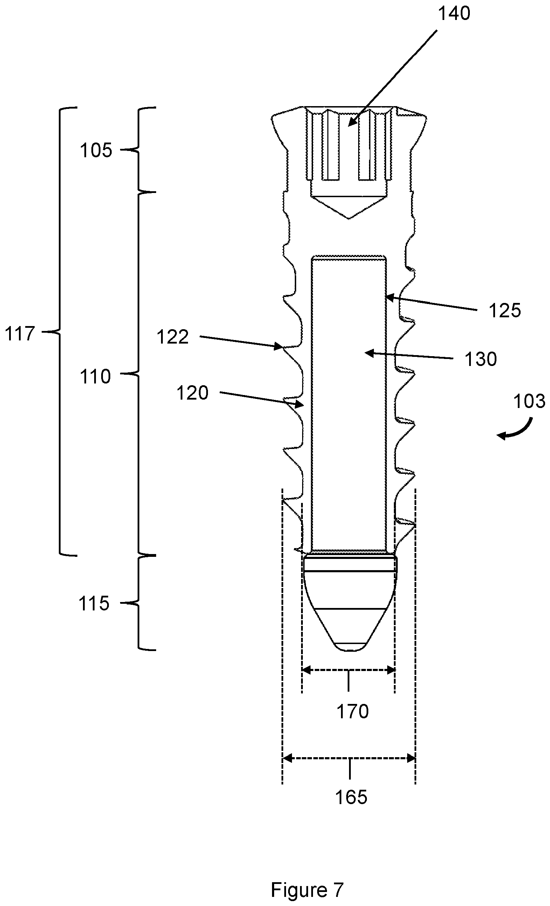

The physical dimensions of the bone screw **100** are generally consistent with the dimensions of screws for insertion in bone that are known in the art. For example, the length **160** may be about 8 to about 100 mm. The outer diameter **165** of the shaft **103**, of which the measurement may include the ends of the threads **122** (for example, see Figure 7), may vary depending on the type of bone in which the screw is being inserted. For example, for insertion in long bones, the screw **100** may have an outer diameter **165** of about 2 mm to about 20 mm, or about 3 mm to about 15 mm, or about 4 mm to about 10 mm. For insertion in vertebrae, the outer diameter **165** may be about 3.5 mm to about 6 mm for cervical vertebrae, and about 4.5 mm to about 9.5 mm for lumbar vertebrae. In some embodiments, the outer diameter **165** may taper at an angle (not shown) of, for instance, about 1 degree or about 10 degrees towards the lower section **115** of the shaft **103**. The inner diameter **170** of the shaft **103**, of which the measurement may not include the ends of the threads **122** (for example, see Figure 7), may also taper at an angle (not shown) of, for example, about 1 degree or about 10 degrees towards the lower section **115** of the shaft **103**. The length and diameter of bore **130** and magnet **135** may also vary depending on the bone in which the screw **100** is being inserted. For instance, for insertion in the vertebral body of a cervical vertebra or a lumbar vertebra, the length (not shown) of bore **130** may be about 10 mm to about 20 mm, while the diameter (not shown) of bore **130** may be 3 to 5 mm for cervical applications. The length and diameter of magnet **135** may conform to the dimensions of bore **130**.

According to embodiments on the invention, bone screw **100** may also include an external textured surface (not shown), which enhances fixation of the bone screw **100** in bone and to aid in screw-bone interface stability. According to certain embodiments, plasma coating of a metal or ceramic may be applied to bone screw **100** to create the external textured surface.

### Disc-shaped Housing

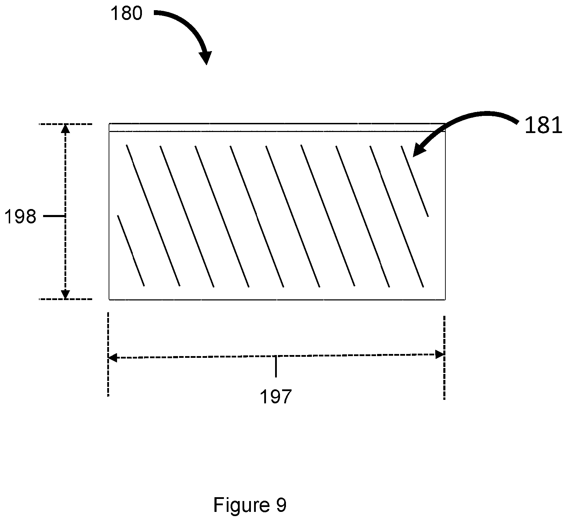

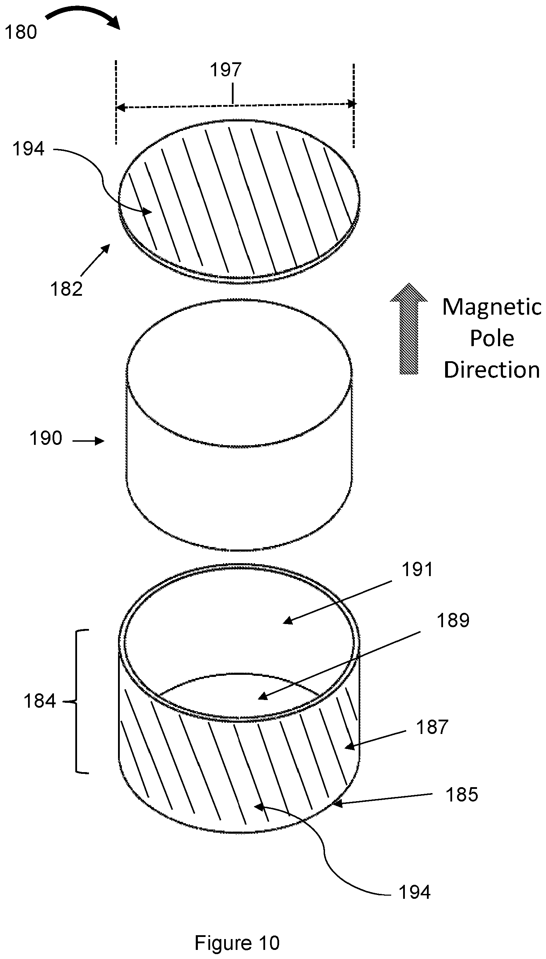

The magnetic device may comprise a disc-shape. In some embodiments, the magnetic device may comprise a disc-shaped housing. With reference to Figures 9 and 10, in certain embodiments, disc-shaped housing **180** may comprise a casing **181** comprising an upper section **182** and a lower section **184**. The disc-shaped housing **180** may comprise a cross-section that is generally circular.

The lower section **184** comprises (i) a base **185**, (ii) an outer wall surface **187**, and (iii) a bore **189** defining an inner wall surface **191**. The upper section **182** comprises a cap. The cap has an outer diameter (not shown) approximately equal to the outer diameter (not shown) of outer wall surface **187**. The upper section **182** is attached to the lower section **184**, for example, by gluing or laser welding, to create a hermetically sealed space. A magnet **190** may be housed in the bore **189**, and may be entirely encased within the disc-shaped housing **180** when the upper section **182** is attached to the lower section **184**. In certain embodiments, the bore **189** may be generally cylindrical in shape, and therefore the magnet **190** in the bore **189** also may be generally cylindrical in shape. Other shapes are also envisioned for the magnet **190** and the bore **189**.

The magnet **190** may be prevented from rotating inside the bore **189**. In some embodiments, the magnet **190** may be prevented from rotating inside the bore **189** by gluing or laser welding the magnet **190** to one or more portions of the inner wall surface **191** of the disc-shaped housing **180**. For example, the magnet may be glued in place with a surgical adhesive such as medical grade epoxy.

The disc-shaped housing **180** may be fabricated with a metal alloy known in the art for orthopaedic applications, for example, titanium, cobalt chromium, or stainless steel. The disc-shaped housing **180** may also include surface modifications **194** such as an external textured surface, which enhances fixation of the disc-shaped housing **180** in bone and to aid in housing-bone interface stability. According to some embodiments, plasma coating of a metal or ceramic is applied to disc-shaped housing **180** to create the external textured surface.

The physical dimensions of disc-shaped housing **180** may vary according to an individual patient's anatomy and the particular bone into which the disc-shaped housing **180** is to be implanted. For example, for implantation in a femur or tibia, the disc-shaped housing **180** may comprise an outer diameter **197** of about 8 mm to about 40 mm, or about 12 mm to about 30 mm, and a height **198** of about 2 mm to about 15 mm, or about 3 mm to about 10 mm. For implantation in vertebrae, the disc-shaped housing **180** may comprise an outer diameter **197** of about 3 mm to about 30 mm, or about 6 mm to about 20 mm, and a height **198** of 2 mm to about 10 mm, or about 3 to about 5 mm.

### Modified Plates

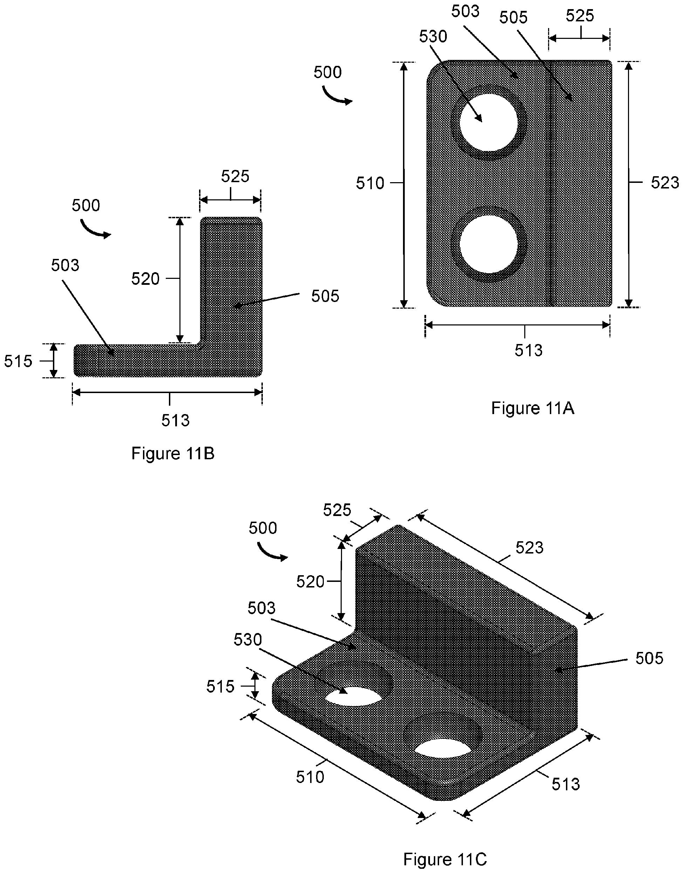

In some embodiments, the magnetic device may be in the shape of a modified plate comprising a plate attached to a casing that encloses the magnet. Figures 11A, 11B, and 11C show a magnetic device **500** in the shape of a plate **503** with a casing **505** that encases the magnet (not shown). The plate **503** in combination with the casing **505** forms a shape resembling the letter "L," with the plate **503** as the horizontal line of the letter and the casing **505** as the vertical line. The plate **503** aspect of the magnetic device **500** may have a length **510** of about 8 mm to about 40 mm, or about 12 mm to about 30 mm; a width **513** of about 5 mm to about 30 mm, or about 8 mm to about 25 mm; and a height **515** of about 1 mm to about 15 mm, or about 2 mm to about 10 mm. The casing **505** encasing the magnet may be in the shape of a rectangular prism that extends from the surface of the plate **503** by a height **520** of about 1 mm to about 25 mm, or about 2 mm to about 20 mm. The length **523** of the casing may be the same as the length **510** of the plate **503** (see Figures 11A, 11B, and 11C), and the width **525** of the casing **505** may be about 1 mm to about 25 mm, or about 2 mm to about 20 mm. The magnetic device **500** may also comprise one or more apertures **530** on the plate **503** that are configured to receive a bone screw or the like. For example, the one or more apertures **530** may be circular and of a diameter appropriate for bone screws known in the art. The number of apertures may vary depending on the type of bone to which the device is being affixed, and is typically from 1 to 6 apertures, i.e., 1, 2, 3, 4, 5, or 6 apertures.

### Bone Plate

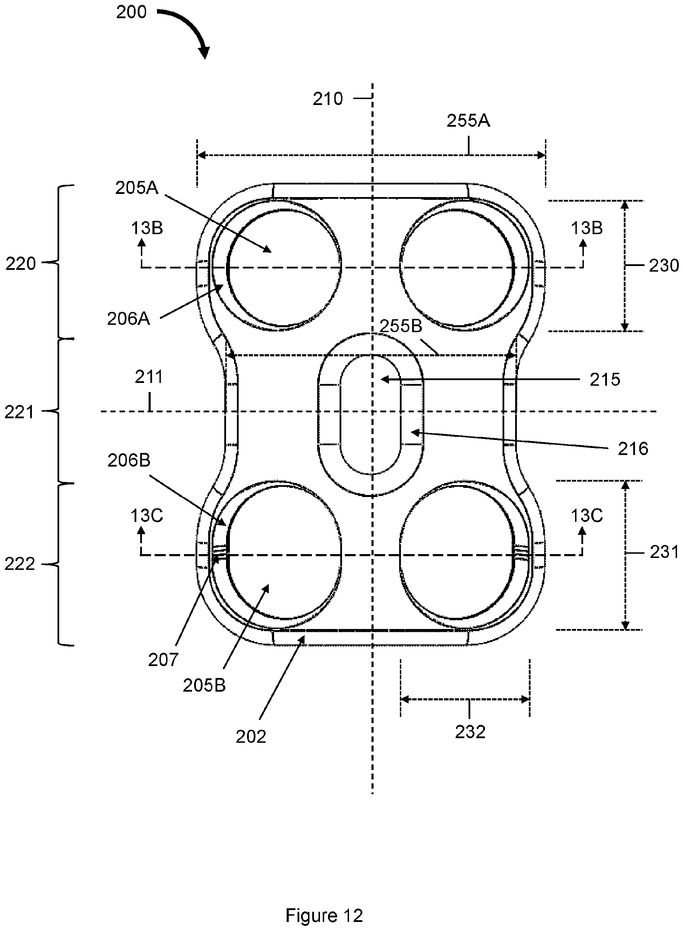

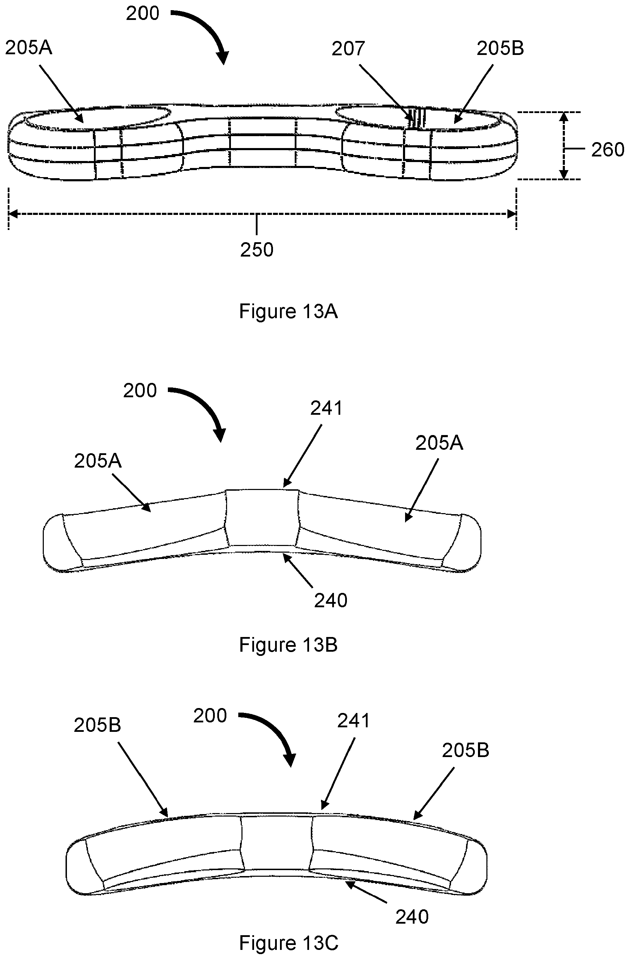

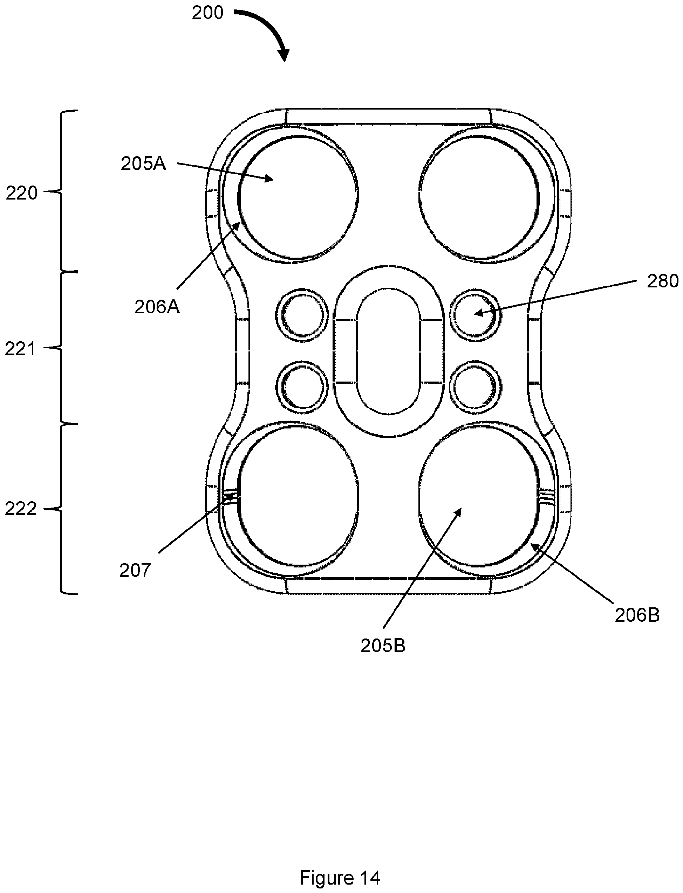

An aspect of the present invention relates to bone plates. With reference to Figures 12, 13A-13C, 14, 15, 16A, and 17B, in certain embodiments, bone plate **200** may comprise a first end section **220**, a middle section second **221**, and a second end section **222**. The bone plate **200** also may comprise a first axis **210** and a second axis **211**.

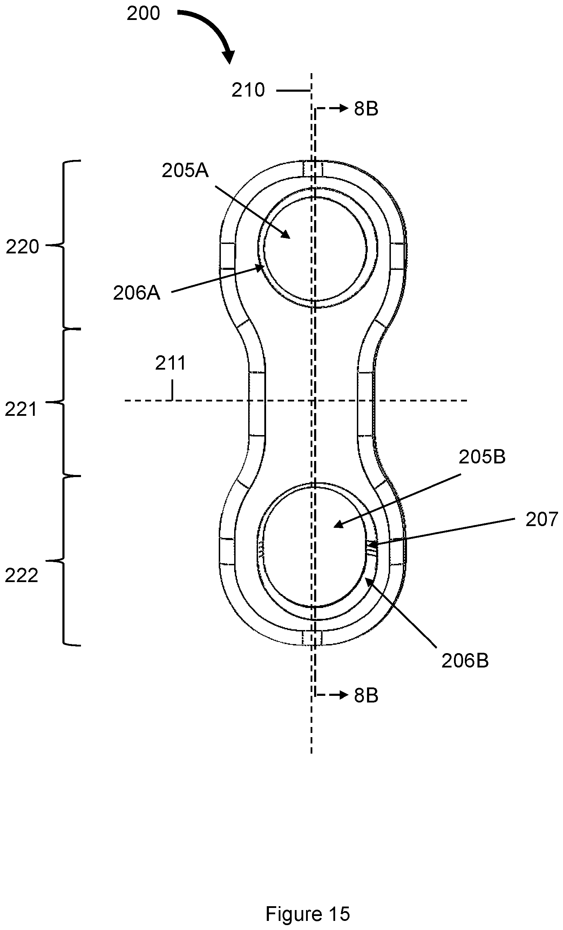

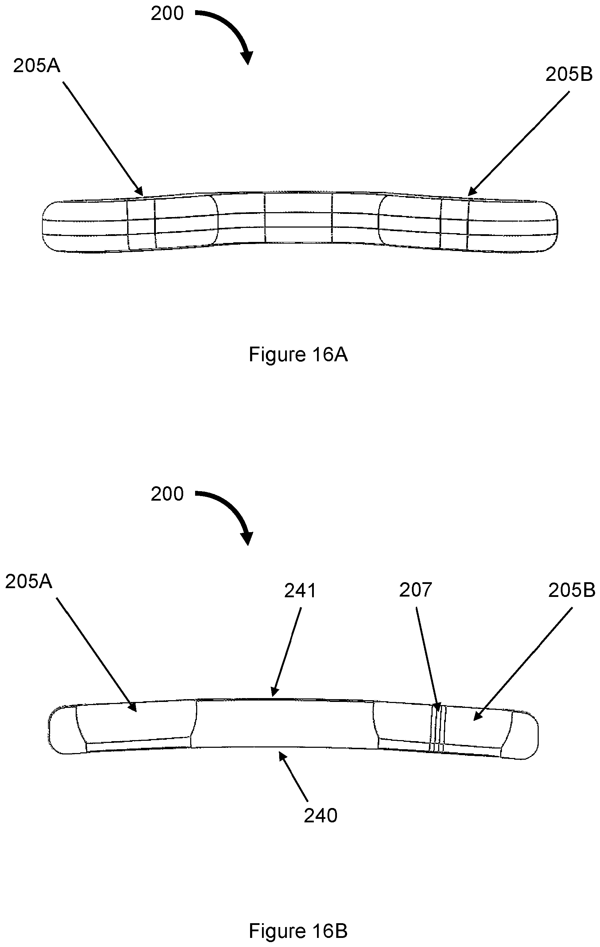

The first end section **220** may comprise at least one aperture **205A**; for instance, the first end section **221** may have one aperture **205A** (as shown in Figures 15, 16A, and 16B), two apertures **205A** (as shown in Figures 12, 13A-13C, and 14), or more than two apertures **205A** (not shown). Each additional aperture **205A** is located along the second axis **211** of the plate **200**. Apertures **205A** of the first end section **220** are generally circular and comprise an inner surface **206A**.

The second end section **222** may comprise at least one aperture **205B**; for instance, the second end section **222** may have one aperture **205B** (as shown in Figures 15, 16A, and 16B), two apertures **205B** (as shown in Figures 12, 13A-13C, and 14), or more than two apertures **205B** (not shown). Each additional aperture **205B** is located along the second axis **211** of the plate **200**. In certain embodiments, the number of apertures **205A** in the first section **220** may be the same as the number of apertures **205B** in the second end section **222**.

The shape of aperture **205B** may comprise an elongated slot, i.e., has ends comprising circular segments and a middle therebetween comprising straight segments that are parallel to each other, such that the aperture **205B** is longer in the direction of the first axis **210** of the plate **200** as compared to the direction of the second axis **211** of the plate **200**. The aperture **205B** may comprise an inner surface **206B** that, in some embodiments, features a marking **207** indicating that the aperture is in the shape of an elongated slot (i.e., that it is not circular). In certain embodiments, the marking **207** may be on a straight segment of the aperture **205B**.

In certain embodiments, aperture **205B** in the second end section **222** may be circular. In such embodiments, the bone plate **200** may comprise a means to decrease its length **250**, such as a means in which the bone plate **200** can collapse in itself or compress (not shown). For example, the first end section **220** and the second end section **222** may be capable of moving towards each other via a first portion of the plate **200** that slides into a second portion of the plate **200.** The first portion, for instance, may be tongue-like in shape, and can slide into the second portion that may comprise a groove or slot. Means to decrease the length **250** of the bone plate **200** are demonstrated by Medtronic's Atlantis Translational^{®} Anterior Cervical Plate, DePuy Synthes's Swift Anterior Cervical Plate, Globulus Medical's Assure Anterior Cervical Plate, K2M's Pyrenees^{®} Translational Cervical Plate System, and NuVasive's Helix Anterior Cervical Plates; and are disclosed in U.S. Patent Nos. 6,306,136, 6,322,562, and 7,666,185.

The middle section **221** may comprise an opening **215** that reduces the weight and amount of material necessary to manufacture the plate **200**. Opening **215** may also be used to hold the plate **200** in place while it is fastened to bone. In some embodiments, opening **215** comprises a fillet **216** around the opening **215**. The opening may be of any shape, including circular, elongated slot, oval, rectangular, square, triangular, and the like.

The middle section **221** may comprise one or more apertures (not shown). In certain embodiments, the number of apertures in the middle section **221** along the second axis **211** is the same as the number of apertures **205A** in the first end section **220** along the second axis **211** and/or the number of apertures **205B** in the second end section **222** along the second axis **211**. In other words, if the apertures **205A** of the first end section **220** and the apertures **205B** of the second end section are each considered as "rows" (as shown in Figure 12), then the middle section may also comprise one or more "rows" of apertures, in which each row (across the first end, middle, and second end sections) have the same number of apertures. In certain embodiments, the apertures in the middle section are in the shape of an elongated slot, similar to apertures **205B** of the second end section **222**.

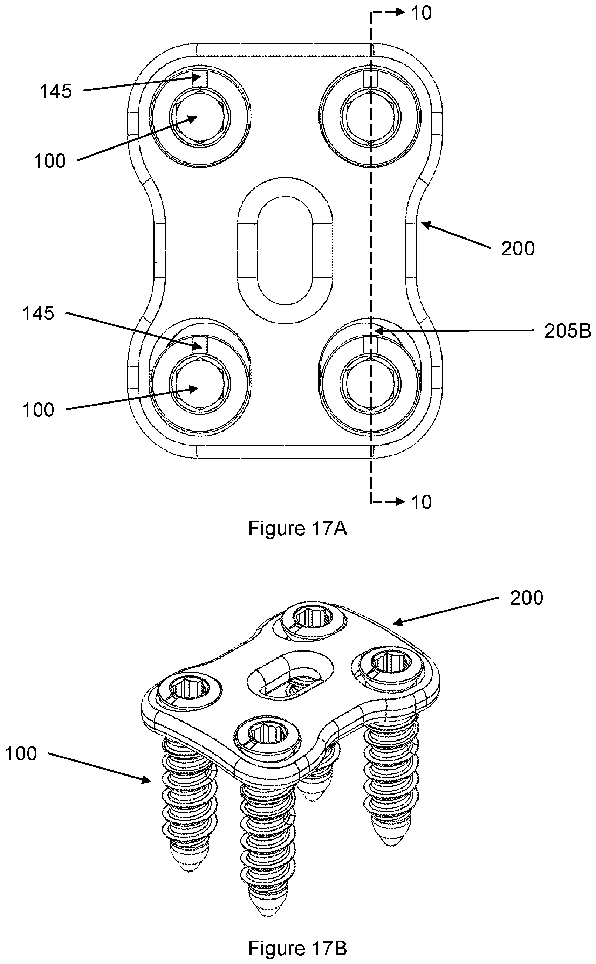

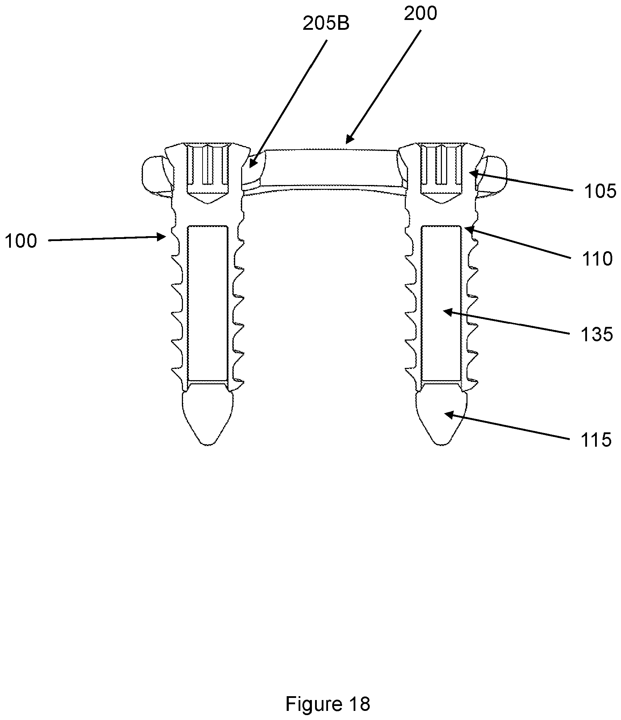

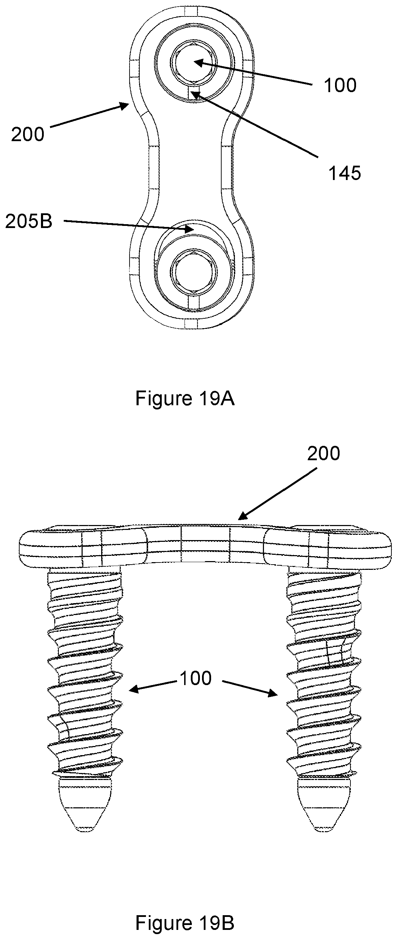

The apertures **205A** of the first end section **220**, the apertures **205B** of the second end section **222**, and any apertures of the middle section **221** all may be configured to accept a bone screw **100** of the invention. Figures 17A, 17B, 18, 19A, and 19B show examples of the bone screw **100** inserted into the apertures.

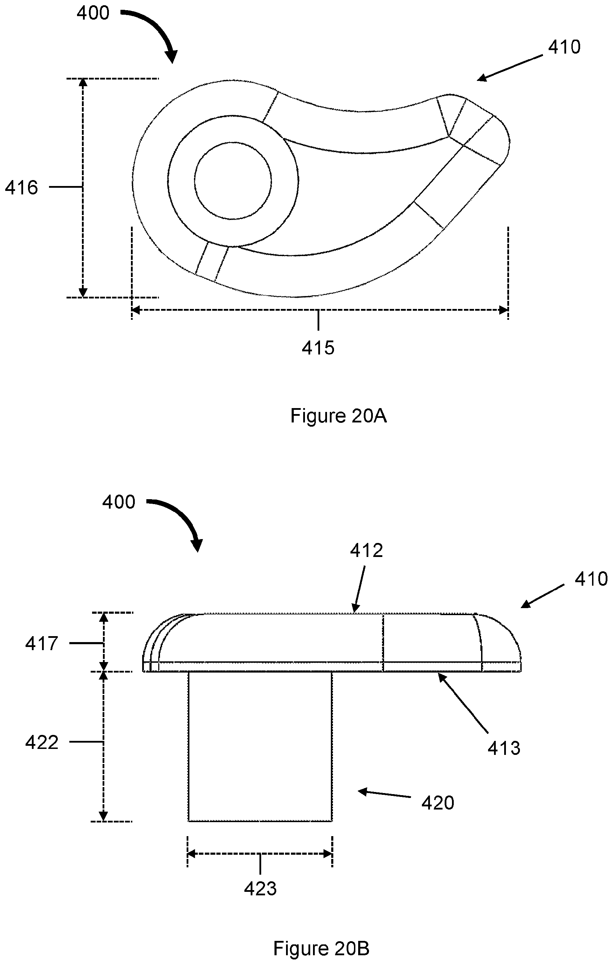

The bone plate **200** may comprise one or more bores **280** that are configured to engage with a locking tab. The locking tab can prevent a bone screw **100** from backing out of the bone once the screw **100** has been inserted into bone through the aperture. An example of a locking tab is shown in Figures 20A and 20B. The locking tab **400** may comprise a head **410** and a stem **420**. The head **410** may comprise a top surface **412** and a bottom surface **413**, in which the bottom surface **413** is relatively flat. The head **410** may be of any shape, including irregular shapes such as the head **410** shown in Figure 20A, which resembles a tear drop. The size of the head **410** (for example, length **415** and width **416**) is limited by the size of the bone plate **200**. For example, the head **410** may be large enough to extend from the bore **280** that will engage with the locking tab **400**, to the bone screw **100** that is inserted through an aperture of the bone plate **200**. But the head **410** may not extend beyond the edges of the bone plate **200**. The thickness **417** of the head **410** may be about 1 to about 10 mm, or about 3 to about 8 mm. The stem **420** is generally cylindrical in shape and is configured to engage with, or be inserted into, the bore **280** of the bone plate **200**. The length **422** of the stem **420** is no greater than the thickness **260** of the bone plate **200**, and the diameter **423** of the stem **420** is configured to the size of the bore **280**.

The bone plate **200** may be generally square or rectangular in shape. In some embodiments, the plate **200** may comprise a generally rounded square or a generally rounded rectangle, i.e., has rounded corners.

In certain embodiments, the plate **200** may comprise edges that are fillet **202**. For example, the fillet may comprise a radius of about 0.5 to about 1 mm, such as about 0.75 mm.

The bone plate **200** may comprise a vertebra-facing surface **240** and an outward-facing surface **241** (i.e., non-vertebra-facing surface). The vertebra-facing surface **240** may be curved along the first axis **210** (equates to the sagittal anatomic plane) and the second axis **211** (equates to the transverse anatomic plane) to configure to the contouring profile of a vertebral body or a portion thereof, or to configure to the vertebral bodies of one or more adjacent vertebrae or portions thereof, such as cervical or lumbar vertebrae. In certain embodiments, the vertebra-facing surface **240** is generally concave. In some embodiments, the outward-facing surface **241** may also be curved, and may be generally convex.

The bone plate **200** may be comprised of materials known in the art for having orthopaedic applications. In certain embodiments, the plate **200** may comprise a uniform material. In some embodiments, the plate **200** may comprise a metal alloy, such as titanium, cobalt-chromium, stainless steel, or a combination thereof. In certain embodiments, the plate **200** may comprise a polymer, such as PEEK or polyurethane, or a combination thereof. In alternative embodiments, the plate **200** may comprise composites of polymers and fibers, such as carbon fiber-reinforced PEEK.

The plate **200** may comprise a length **250** that is dependent on the number of apertures in the direction of the first axis that is in the middle section **221**. For example, if there are no apertures in the middle section **221**, the length **250** of the plate **200** may be about 10 to 50 mm. If there is one "row" of one or more apertures in the middle section, i.e., at least one aperture along the first axis **210** in the middle section **221**, then the length **250** of the plate **200** may be about 30 mm to about 60 mm. If there are two "rows" of one or more apertures in the middle section **221**, i.e., two apertures along the first axis **210**, then the length **250** of the plate **200** may be about 45 mm to about 90 mm. The length **250** of the plate **200** is proportionally longer if there are three "rows" of apertures, four "rows" of apertures, etc.

The width **255A** of the first end section **220** and the second end section **222** of the plate **200** may be the same. The width **255A** may be about 12 mm to about 20 mm for applications relating to cervical vertebrae, and about 8 mm to about 20 mm for applications relating to thoracic/lumbar vertebrae. In certain embodiments, the width **255B** of the middle section **221** may be the same as the width **255A**, such as if the middle section **221** comprises one or more apertures. In some embodiments, the width **255B** of the middle section **221** may be less than the width **255A** of the first section **220** or the second section **222**, such as if the middle section **221** does not comprise any apertures; in these embodiments, the width **255B** of the middle section may be about 10 mm to about 18 mm for applications relating to cervical vertebrae, or about 8 mm to about 20 mm for applications relating to thoracic/lumbar vertebrae.

The thickness **260** of the plate **200** may vary according to the type of vertebrae in which the plate **200** will be attached. For example, for attachment to the vertebral bodies of cervical vertebrae, the thickness **260** may be about 1 mm to about 5 mm; for attachment to the vertebral bodies of thoracic or lumbar vertebrae, the thickness **260** may be about 1 mm to about 6 mm.

The size of the apertures may accommodate the bone screws **100** of the present invention. For example, the diameter **230** of the aperture **205A** may be about 4 to about 6 mm for a plate **200** that will be attached to the vertebral bodies of cervical vertebrae, and about 4.5 to about 9.5 mm for a plate **200** that will be attached to the vertebral bodies of thoracic/lumbar vertebrae. The width **232** (measured in the direction of the second axis **211** of the plate **200**) of the aperture **205B** may be about 4 to about 6 mm for a plate **200** that will be attached to the vertebral bodies of cervical vertebrae, and about 4.5 to about 9.5 mm for a plate **200** that will be attached to the vertebral bodies of thoracic/lumbar vertebrae. The length **231** (measured in the direction of the first axis of the plate **200**) of the aperture **205B** may be about 5 to about 7 mm for a plate **200** that will be attached to the vertebral bodies of cervical vertebrae, and about 5 to about 11 mm for a plate **200** that will be attached to the vertebral bodies of thoracic/lumbar vertebrae.

### Use of the Magnetic Devices in a Knee Joint

In one aspect, the methods comprise implanting one or more magnetic devices into the femur and one or more magnetic devices into the tibia. The magnetic devices may be oriented such that a repulsive force is generated between the magnetic device(s) in the femur and the magnetic device(s) in the tibia. The implantation may comprise preparing one or more bores in the femur and one or more bores in the tibia for each of the magnetic devices that are being implanted, and then affixing the magnetic devices in the bores in the orientation that results in the repulsive force between the magnetic device(s) in the femur and the magnetic device(s) in the tibia. The bore may comprise a shape that is compatible with the shape of the magnetic device, e.g., a cylindrical bore for implantation of cylindrical magnetic devices, a bore in the shape of a disc or a portion of a disc for implantation of disc-shaped magnetic devices, etc. The bore may be created using a bone drill, bone saw, or other devices known in the art for use in orthopaedic surgeries or procedures.

The magnetic device(s) may be implanted in the distal femur, such as in the medial condyle or the lateral condyle, or both the medial condyle and the lateral condyle. For embodiments in which the magnetic device(s) is implanted in the medial condyle, the bore(s) may be prepared on the medial surface, the anterior surface, the posterior surface, or a combination thereof, preferably the medial surface, of the medial condyle. For embodiments in which the magnetic device(s) is implanted in the lateral condyle, the bore(s) may be prepared on the lateral surface, the anterior surface, the posterior surface, or a combination thereof, preferably the lateral surface, of the medial condyle.

The magnetic device(s) may be implanted in the proximal tibia, such as in the medial condyle or the lateral condyle, or both the medial condyle and the lateral condyle. For embodiments in which the magnetic device(s) is implanted in the medial condyle, the bore(s) may be prepared on the medial surface, the anterior surface, the posterior surface, or a combination thereof, preferably the medial surface, of the medial condyle. For embodiments in which the magnetic device(s) is implanted in the medial condyle, the bore(s) may be prepared on the lateral surface, the anterior surface, the posterior surface, or a combination thereof, preferably the lateral surface, of the lateral condyle.

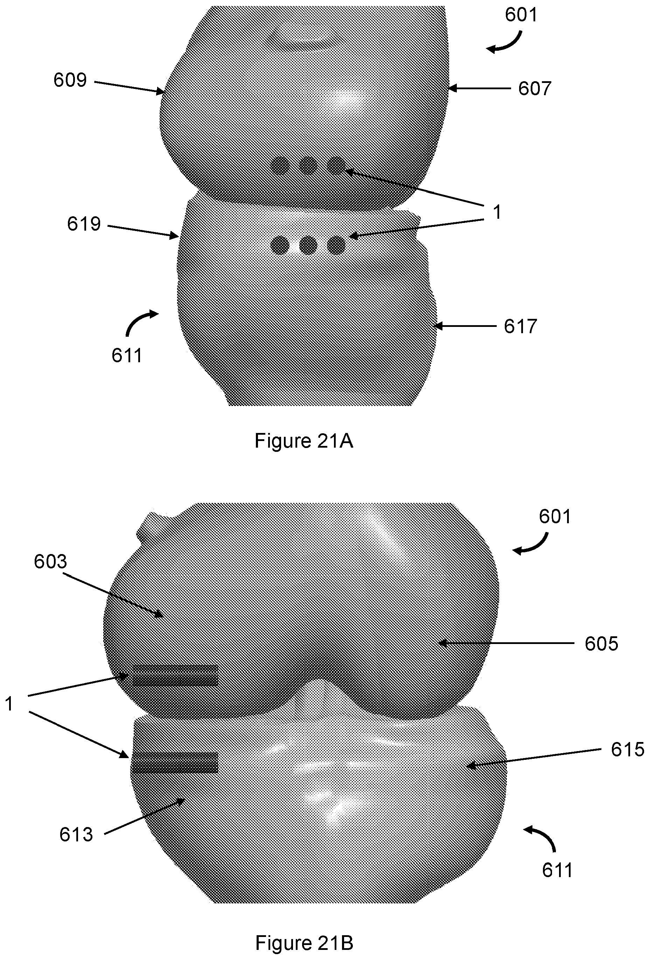

Implanted magnetic devices in the femur and tibia are exemplified in Figures 21A-30B. These figures illustrate magnetic device configurations for implantation in a femur **601** having a medial condyle **603** and a lateral condyle **605**, along with an anterior surface **607** and a posterior surface **609**; and a tibia **611** having a medial condyle **613** and a lateral condyle **615**, and an anterior surface **617** and a posterior surface **619**. These figures demonstrate magnetic devices that are cylindrical **1**, rectangular prism-shaped **50**, or disc-shaped **180**, or that are bone screws **100** or bone plates **500**.

The number of magnetic devices implanted in the femur may range from one to five, e.g., one, two, three, four, or five magnetic devices. In some embodiments, the number of magnet devices implanted in the tibia may range from one to five, e.g., one, two, three, four, or five magnetic devices. The number of magnetic devices implanted in the femur may be the same as the number of magnetic devices implanted in the tibia; such a configuration is illustrated in Figures 21A and 21B, in which three cylindrical magnetic devices **1** are implanted into the medial condyle **603** of the femur **601**, and three cylindrical magnetic devices **1** are implanted in the medial condyle **613** of the tibia **611**.

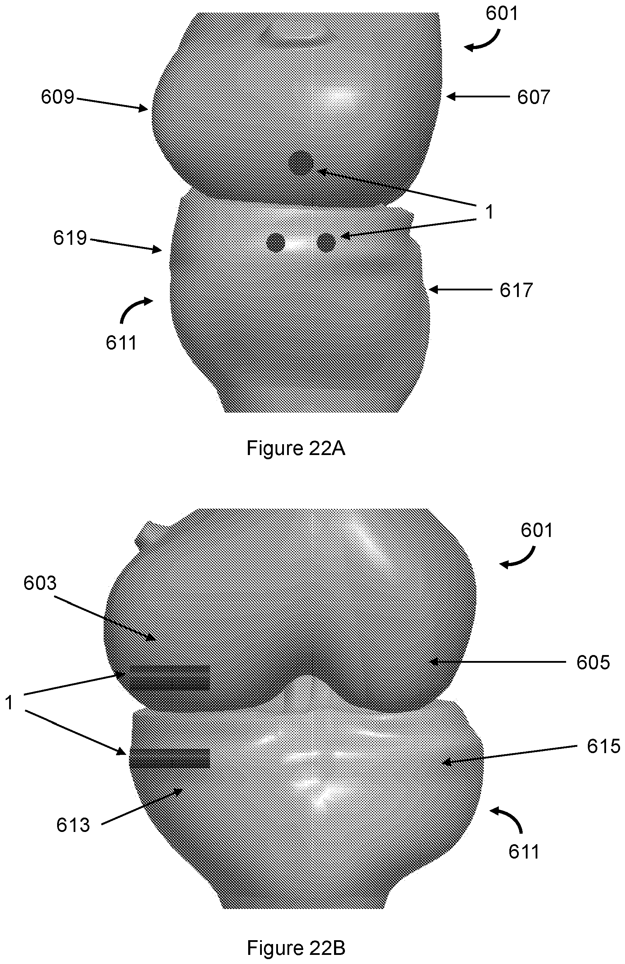

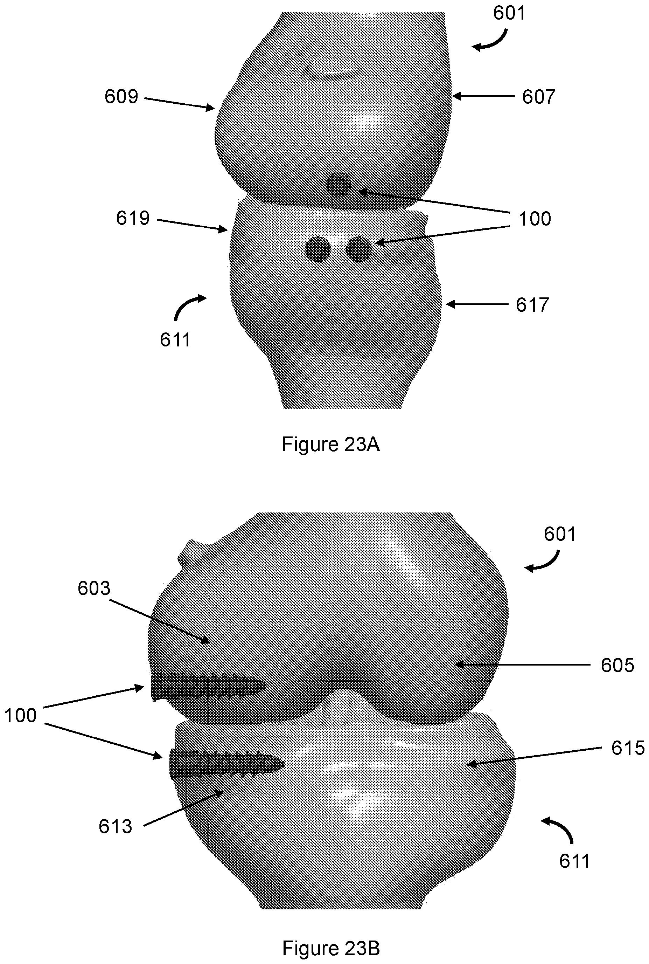

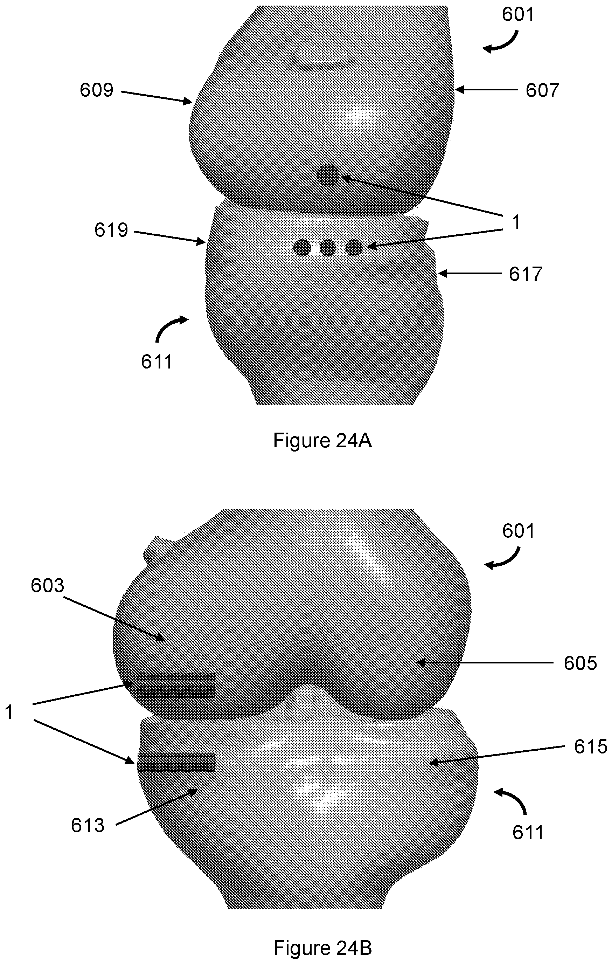

Alternatively, the number of magnets implanted in the femur may be different than the number of magnets implanted in the tibia. Figures 22A and 22B show one cylindrical magnetic device **1** implanted into the medial condyle **603** of the femur **601**, and two cylindrical magnetic devices **1** implanted into the medial condyle **613** of the tibia **611**. Figures 23A and 23B show one bone-screw magnetic device **100** implanted into the medial condyle **603** of the femur **601**, and two bone-screw magnetic devices **100** implanted into the medial condyle **613** of the tibia **611**. Further, Figures 24A and 24B show one cylindrical magnetic device **1** implanted into the medial condyle **603** of the femur **601**, and three cylindrical magnetic devices **1** implanted into the medial condyle **613** of the tibia **611**.

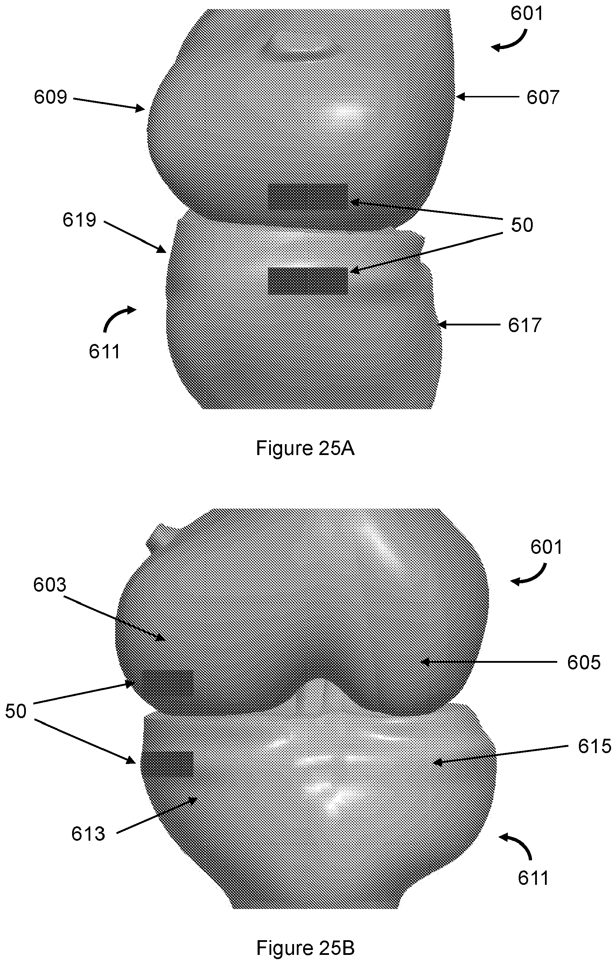

The shape of the magnetic device(s) implanted in the femur may be the same as the shape of the magnetic device(s) implanted in the tibia, as shown in Figures 21A, 21B, 22A, 22B, 24A, and 24B, in which the magnetic devices are all cylindrical magnetic devices **1**, or as shown in Figures 23A and 23B, in which the magnetic devices in both the femur and tibia are bone screws **100**. In addition, in Figures 25A and 25B, rectangular prism-shaped magnetic devices **50** may be implanted into the medial condyle **603** of the femur **601** and the medial condyle **613** of the tibia **611**.

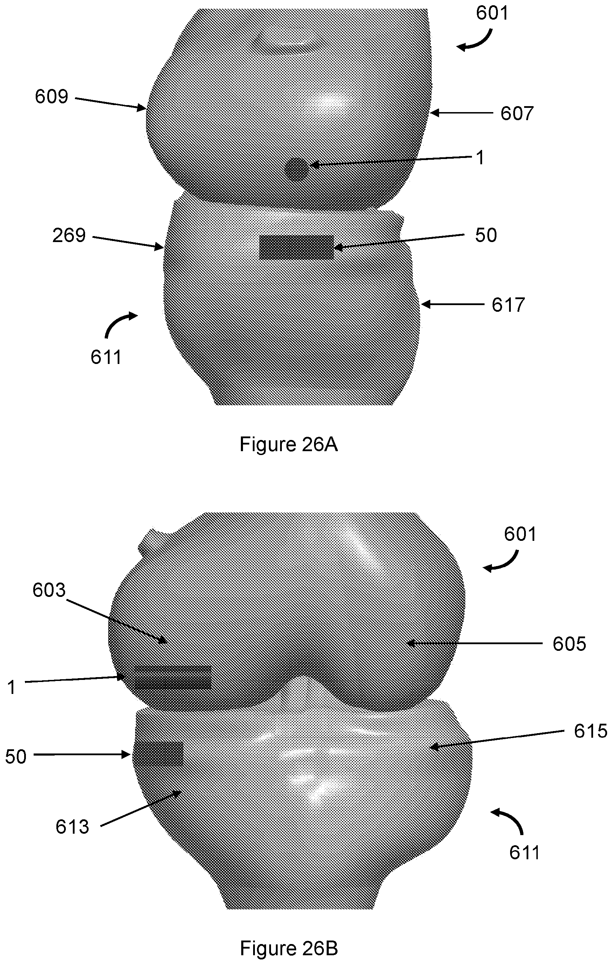

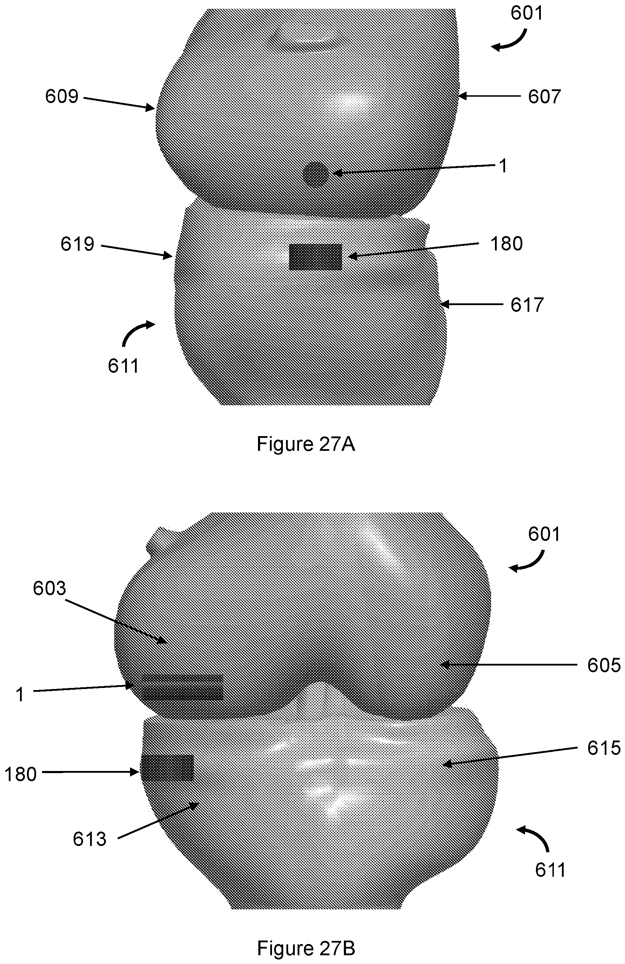

The magnetic device(s) implanted in the femur may be a different shape than the magnetic device(s) implanted in the tibia. For instance, as shown in Figures 26A and 26B, a cylindrical magnetic device **1** may be implanted into the medial condyle **603** of the femur **601** and a rectangular prism-shaped magnetic device **50** may be implanted into the medial condyle **613** of the tibia **611**. And as shown in Figures 27A and 27B, a cylindrical magnetic device **1** may be implanted into the medial condyle **603** of the femur **601** and a disc-shaped magnetic device **180** may be implanted into the medial condyle **613** of the tibia **611**.

The magnetic devices are implanted in a configuration such that the one or more magnetic devices of the femur are maintained at a prescribed distance, or a range of distances, from the one or more magnetic devices of the tibia while the knee undergoes motion. The distance may be about 10 mm to about 60 mm, or about 15 mm to about 50 mm. The chosen configuration may also take into account the patient anatomy and the desired level of unloading and stability of the knee joint. For example, if a greater level of unloading is desired, the magnetic devices may be implanted such that they are closer together, or stronger or larger magnets are used.

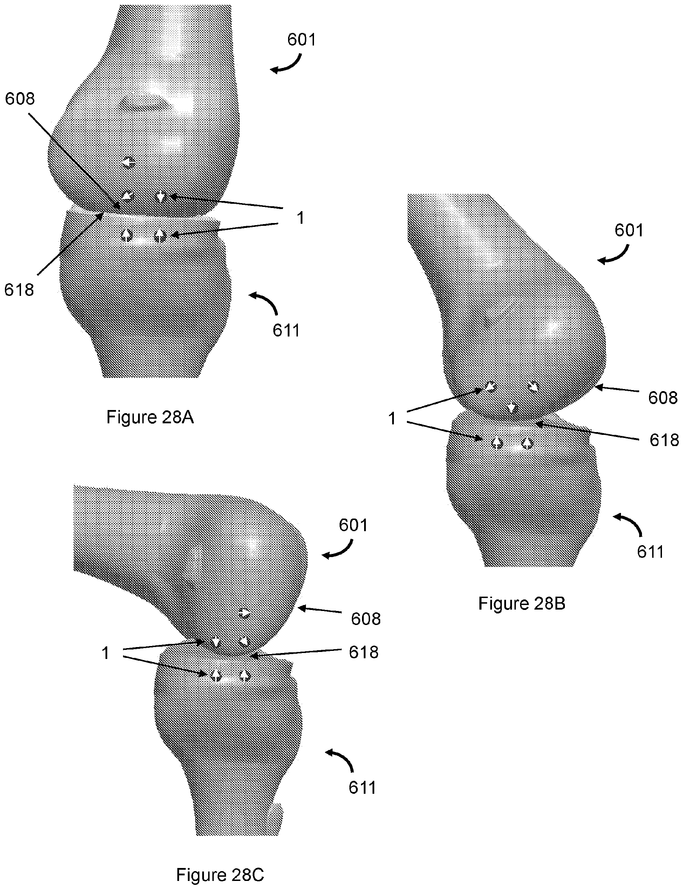

From a side perspective, the magnetic devices may be implanted into the femur and tibia such that the magnetic device(s) in the femur and the magnetic device(s) in the tibia are each in a linear configuration, in which the linear configuration of the magnetic device(s) in the femur is parallel to the linear configuration of the magnetic device(s) in the tibia when the knee is in an extended position, i.e., a flexion angle of 0° *(see, e.g.,* Figures 21A, 25A). Alternatively, the magnetic device(s) may be implanted such that the magnetic device(s) in the femur and/or tibia are in a curved configuration. The curved configuration may generally correspond with the curvature of the surface of the bone(s), such as the curvature of the surface of the condyle(s) of the femur or the curvature of the surface of the condyle(s) of the tibia. For example, magnetic device(s) implanted into the medial surface of the medial condyle of the femur may be in a configuration that generally corresponds with the anterior-posterior curvature of the distal surface of the medial condyle of the femur, as depicted in Figures 28A-28C and 29A-29C. Magnetic device(s) implanted into the medial surface of the medial condyle of the tibia may be in a configuration that generally corresponds with the anterior-posterior curvature of the proximal surface of the medial condyle of the tibia (not shown). Alternatively or in addition, magnetic device(s) implanted into the lateral surface of the lateral condyle of the femur may be in a configuration that generally corresponds with the anterior-posterior curvature of the distal surface of the lateral condyle of the femur. Magnetic device(s) implanted into the lateral surface of the lateral condyle of the tibia may be in a configuration that generally corresponds with the anterior-posterior curvature of the proximal surface of the lateral condyle of the femur and/or tibia. The magnetic device(s) may be implanted at a prescribed distance, or range of distances, from the surface of the bone, in which the distance is measured from magnetic device(s) to the closest point on the bone surface, of about 10 mm to about 60 mm, or about 15 mm to about 50 mm. In some embodiments, the magnetic device(s) implanted in the femur and the magnetic device(s) implanted in the tibia may be in different configurations. For example, the magnetic device(s) implanted in the femur may be in a curved configuration and the magnetic device(s) implanted in the tibia may be in a linear configuration, or vice versa.

Figures 28A-28C and 29A-29C illustrate embodiments in which magnetic device(s) **1** implanted in the femur **601** are in a curved configuration and magnetic device(s) **1** (Figures 28A-28C) or **50** (Figures 29A-29C) implanted in the tibia **611** are in a linear configuration. The curved configuration of magnetic devices **1** in the femur **601** generally corresponds with the anterior-posterior curvature of the distal surface 608 of the medial condyle of femur **601** that is in contact (through cartilage) with the proximal surface **618** of the medial condyle of tibia **611**.

The linear configuration of magnetic devices may be achieved by using two or more magnetic devices, such as two or more cylindrical magnetic devices **1** (*see, e.g.,* Figures 21A, 28A-28C) or two or more bone screw magnetic devices **100** (*see, e.g.,* Figure 23A), or the linear configuration may be achieved by using a single magnetic device, such as a rectangular prism magnetic device **50** *(*s*ee, e.g.,* Figures 25A, 16A, 29A-29C) or a disc-shaped magnetic device **180** (*see, e.g.,* Figure 27A). The curved configuration of magnetic devices may be achieved by using two or more magnetic devices, such as two or more cylindrical magnetic devices **1** (*see,* e.g., Figures 28A-28C, 29A-29C) or two or more bone screw magnetic devices **100,** or the curved configuration may be achieved by using a single magnetic device that is curved in shape (not shown).

In some embodiments, the magnetic device(s) in the femur will be centered in an anterior-posterior direction with the magnetic device(s) in the tibia.

In another aspect, the methods may involve attaching magnetic device(s) to the surface of the femur and the tibia. For instance, the magnetic devices may be attached to the surface of the medial condyle of the femur and of the tibia. Without being bound by theory, the plates can generate a greater repulsive force, because the external magnets can be placed closer to each other relative to the anatomically-constrained distances of those placed within the femur and the tibia.

These methods may comprise affixing magnetic device(s) to the surface of the femur and affixing magnetic device(s) to the surface of the tibia, and orienting the magnetic devices such that a repulsive force is generated between the magnetic device(s) affixed to the femur and the magnetic device(s) affixed to the tibia. The magnetic devices may be affixed to the bone surfaces using bone screws, bone cement, or other means of adhering devices to bone surfaces that are known in the art. Once affixed to the surface of the femur and tibia, the magnetic device(s) may be covered by the soft tissues and skin of the knee.

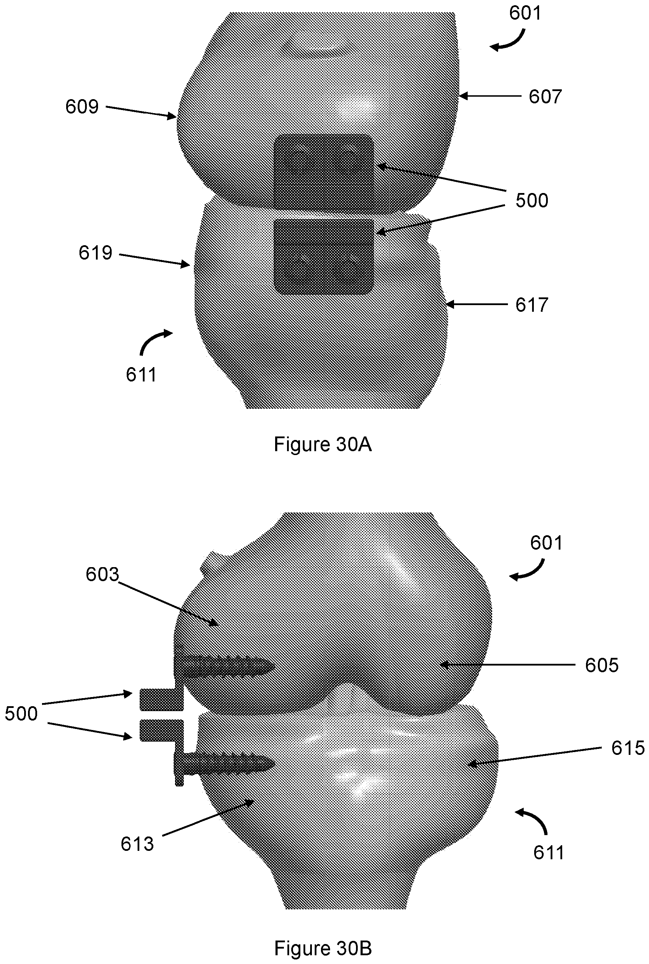

The magnetic device(s) affixed to the surface of the femur and tibia may be a magnetic device **500** comprising a plate **503** with a casing **505** that encases the magnet, as described above and as shown in Figures 11A, 11B, and 11C. These plates affixed to the surface of the femur and tibia is illustrated in Figures 30A and 30B, which shows the plate-like magnetic device **500** attached to the medial condyle **603** of the femur **601** and a plate-like magnetic device **500** attached to the medial condyle **613** of the tibia **611**. The magnets and the casing that encases them may be rectangular (as shown in Figures 11A, 11B, and 11C) or, alternatively, may be curved or shaped as an arc (not shown) so that the distance between the magnet of the plate affixed to the femur and the magnet of the plate affixed to the tibia is generally constant; this distance may be about 2 mm to about 30 mm, or about 5 mm to about 25 mm.

### Use of the Magnetic Devices in an Acetabulofemoral Joint

In one aspect, the methods comprise implanting one or more magnetic devices in the femur and one or more magnetic devices in the hip bone to reduce loading across the acetabulofemoral joint. The magnetic devices may be oriented such that a repulsive force is generated between the magnetic device(s) of the femur and the magnetic device(s) of the hip bone. The implantation may comprise preparing one or more bores in the femur and one or more bores in the hip bone for each of the magnetic devices that are being implanted, and then affixing the magnetic devices in the bores in the orientation that results in the repulsive force. The bore may comprise a shape that is compatible with the shape of the magnetic device, e.g., a cylindrical bore for implantation of cylindrical magnetic devices, a bore in the shape of a disc or a portion of a disc for implantation of disc-shaped magnetic devices, etc. The bore may be created using a bone drill, bone saw, or other devices known in the art for use in orthopaedic surgeries or procedures.

For implantation in the femur, the magnetic device(s) may be implanted in the proximal femur, such as in the femoral head. For implantation in the hip bone, the magnetic device(s) may be implanted adjacent to the acetabulum.

The number of magnetic devices implanted in the femur and the hip bone may each range from one to five, e.g., one, two, three, four, or five magnetic devices. The number of magnetic devices implanted in the femur may be the same as the number of magnetic devices implanted in the hip bone, or the number of magnets implanted in the femur may be different than the number of magnets implanted in the hip bone. Further, the shape of the magnetic device(s) implanted in the femur may be the same or different than the shape of the magnetic device(s) implanted in the hip bone.

The magnetic devices are implanted in a configuration such that the one or more magnetic devices of the femur are maintained at a prescribed distance, or a range of distances, from the one or more magnetic devices of the hip while the acetabulofemoral joint undergoes motion. The chosen array may also take into account the patient anatomy and the desired level of unloading and stability of the acetabulofemoral joint. In some embodiments, the magnetic devices may be implanted such that the magnetic device(s) in the femur and the magnetic device(s) in the hip bone are each in a linear configuration, in which the linear configuration of the magnetic device(s) in the femur is parallel to the linear configuration of the magnetic device(s) in the hip bone. Alternatively, the magnetic device(s) may be implanted such that the magnetic device(s) in the femur and/or hip bone are in a curved configuration. The curved configuration may generally correspond with the curvature of the surface of the bone(s), such as the curvature of the femoral head of the femur or the curvature of the acetabulum of the hip bone. In certain embodiments, the magnetic device(s) in the femur and the magnetic device(s) in the hip bone are in different configurations; for instance, the magnetic device(s) in the femur may be in a curved configuration while the magnetic device(s) in the hip bone are in a linear configuration, or vice versa.

### Use of the Magnetic Devices in an Ankle Joint

In one aspect, the methods comprise implanting one or more magnetic devices in the distal tibia and/or fibula and one or more magnetic devices in the talus, wherein the one or more magnetic devices in the tibia and/or fibula and the one or more magnetic devices in the talus are oriented to generate a repulsive magnetic force therebetween. In some embodiments, one or more magnetic devices are implanted in both the tibia and the fibula. In other embodiments, one or more magnetic devices are implanted in the tibia only. In certain embodiments, one or more magnetic devices are implanted in the proximal talus.

In one aspect, the methods of the present invention involve implanting one or more magnetic devices in the tibia and/or fibula, and one or more magnetic devices in the talus to reduce loading across the ankle joint. The magnetic devices may be oriented such that a repulsive force is generated between the magnetic device(s) of the tibia and/or fibula, and the magnetic device(s) of the talus. The implantation may comprise preparing one or more bores in the tibia and/or fibula, and one or more bores in the talus for each of the magnetic devices that are being implanted, and then affixing the magnetic devices in the bores in the orientation that results in the repulsive force. The bore may comprise a shape that is compatible with the shape of the magnetic device, e.g., a cylindrical bore for implantation of cylindrical magnetic devices, a bore in the shape of a disc or a portion of a disc for implantation of disc-shaped magnetic devices, etc. The bore may be created using a bone drill, bone saw, or other devices known in the art for use in orthopaedic surgeries or procedures.

For implantation in the tibia and/or fibula, the magnetic device(s) may be implanted in the distal tibia and/or fibula. The magnetic devices may be implanted in the tibia only, the fibula only, or both the tibia and the fibula. For implantation in the talus, the magnetic device(s) may be implanted in the proximal talus. The magnetic devices may be implanted into the medial surface of the tibia and/or fibula, and the medial surface of the talus. Alternatively, the magnetic devices may be implanted into the lateral surface of the tibia and/or fibula, and the lateral surface of the talus. In some embodiments, the magnetic devices may be implanted into both the medial and lateral surfaces of the tibia and/or fibula, and both the medial and lateral surfaces of the talus.

The number of magnetic devices implanted in the tibia and/or fibula and in the talus may each range from one to five, e.g., one, two, three, four, or five magnetic devices. The number of magnetic devices implanted in the fibula and/or tibia may be the same as the number of magnetic devices implanted in the talus, or the number of magnets implanted in the tibia and/or fibula may be different than the number of magnets implanted in the talus. If the magnetic device(s) are implanted in both the tibia and the fibula, the number of magnetic devices implanted in the tibia may be the same or different than the number of magnetic devices implanted in the fibula. Further, the shape of the magnetic device(s) implanted in the tibia and/or fibula may be the same or different than the shape of the magnetic device(s) implanted in the talus. If the magnetic device(s) are implanted in both the tibia and the fibula, the shape of the magnetic device(s) implanted in the tibia may be the same or different than the shape of the magnetic device(s) implanted in the fibula.

The magnetic devices are implanted in a configuration such that the one or more magnetic devices of the tibia and/or fibula are maintained at a prescribed distance, or a range of distances, from the one or more magnetic devices of the talus while the ankle joint undergoes motion. The chosen array may also take into account the patient anatomy and the desired level of unloading and stability of the ankle joint. In some embodiments, the magnetic devices may be implanted such that the magnetic device(s) in the tibia and/or fibula and the magnetic device(s) in the talus are each in a linear configuration, in which the linear configuration of the magnetic device(s) in the tibia and/or fibula is parallel to the linear configuration of the magnetic device(s) in the talus. Alternatively, the magnetic device(s) may be implanted such that the magnetic device(s) in the tibia and/or fibula, and/or in the talus are in a curved configuration. The curved configuration may generally correspond with the curvature of the surface of the bone(s), such as the anterior-posterior curvature of the distal surface of the tibia and/or fibula or the anterior-posterior curvature of the proximal surface of the talus. In certain embodiments, the magnetic device(s) in the tibia and/or fibula and the magnetic device(s) in the talus are in different configurations; for instance, the magnetic device(s) in the tibia and/or fibula may be in a curved configuration while the magnetic device(s) in the talus are in a linear configuration, or vice versa.

### Use of the Magnetic Devices in the Intervertebral Joint

In one aspect, the methods comprise implanting one or more magnetic devices in a first vertebra that is superior to an intervertebral joint and one or more magnetic devices in a second vertebra that is inferior to an intervertebral j oint to reduce loading across the intervertebral joint. The magnetic devices may be oriented such that a repulsive force is generated between the magnetic device(s) of the first vertebra and the magnetic device(s) of the second vertebra bone.

The implantation of the magnetic device(s) in the first vertebra and in the second vertebra may exert a traction force on the vertebrae, such as a consistent, continuous, low magnitude traction force that can maintain disc height and health and relieves acute and chronic neck pain. Implantation of the magnetic device(s) in the first vertebra and in the second vertebra may also treat pain caused by a herniated disk between the first vertebra and the second vertebra, and/or it may treat a herniated disk between the first vertebra and the second vertebra. In certain embodiments, implantation of the magnetic device(s) in the first vertebra and in the second vertebra may relax soft tissue and separate spine vertebral segments by imparting magnetic force(s) to separate and levitate cervical spine segments.

The implantation may comprise preparing one or more bores in the first vertebra and one or more bores in the second vertebra for each of the magnetic devices that are being implanted, and then affixing the magnetic devices in the bores in the orientation that results in the repulsive force. The bore may comprise a shape that is compatible with the shape of the magnetic device, e.g., a cylindrical bore for implantation of cylindrical magnetic devices, a bore in the shape of a disc or a portion of a disc for implantation of disc-shaped magnetic devices, etc. The bore may be created using a bone drill, bone saw, or other devices known in the art for use in orthopaedic surgeries or procedures.

The magnetic device(s) may be implanted in the vertebral body of the first vertebra and in the vertebral body of the second vertebra. In some embodiments, the bore to implant the magnetic devices may be prepared on the anterior surface of the vertebral bodies.

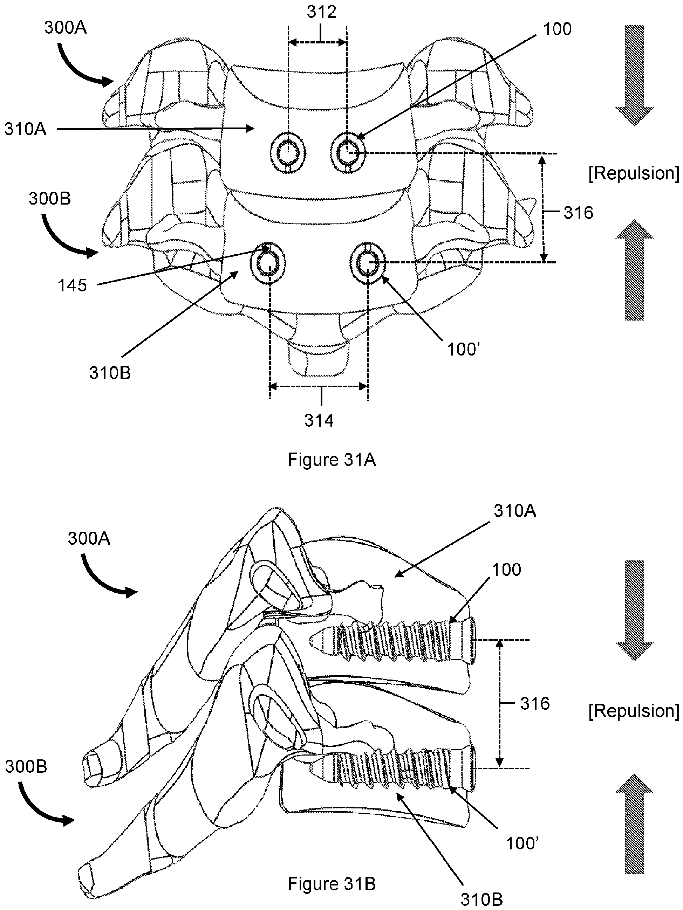

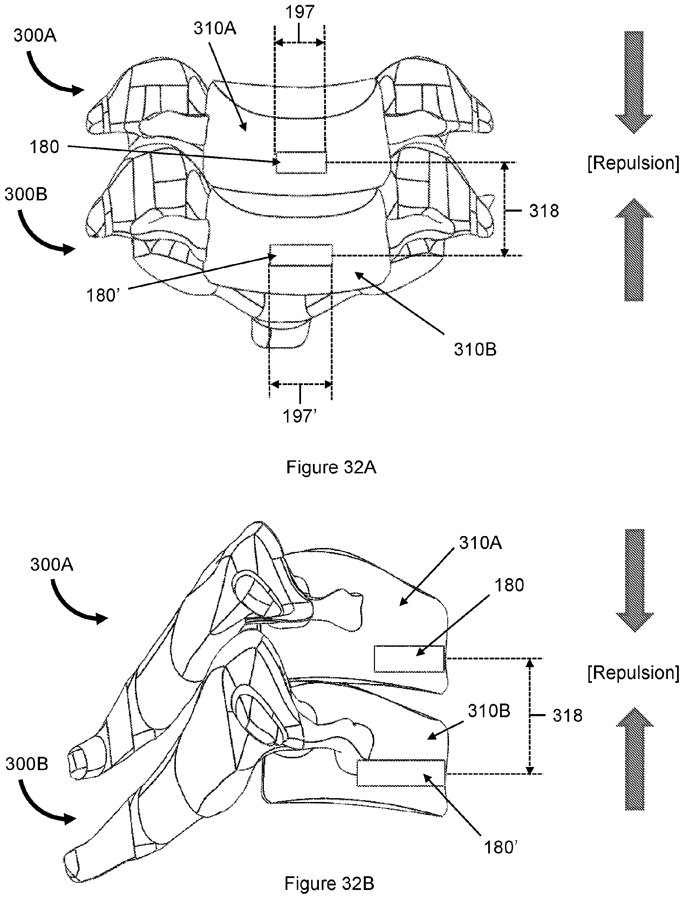

Implanted magnetic devices in vertebrae are exemplified in Figures 31A, 31B, 32A, and 32B. These figures illustrate a plurality of bone screws **100/100'** and disc-shaped housings **180/180'** implanted in the vertebral body **310A** of a first superior vertebra **300A** and in the vertebral body **310B** of a second, adjacent inferior vertebra **300B,** according to certain embodiments of the invention.

In certain embodiments of the invention, bone screws **100** and bone screws **100'** are inserted in each of vertebral bodies **310A** and **310B,** respectively, of vertebrae **300A** and **300B**, respectively. The marks **145** are used to orient the bone screws **100**/**100'** such that the polarities of the magnets within the bone screws **100**/**100'** generate a repulsive force between the magnets (not shown in Figures 31A or 31B) of the bone screws **100** and the magnets (not shown in Figures 31A or 31B) of the bone screws **100'** fastened to vertebrae **300A** and **300B,** respectively.

The number of magnetic devices implanted in the vertebrae range from one to five, e.g., one, two, three, four, or five magnetic devices. According to embodiments of the invention, and as demonstrated in Figures 31A and 31B, two bone screws **100** may be implanted in vertebral body **310A** of vertebra **300A** such that their longitudinal centers are separate by a distance **312**. The distance **312** can vary depending upon the individual anatomy of the patient. For example, distance **312** can be about 8 mm to about 12 mm. Two bone screws **100'** are also implanted in vertebral body **310B** of vertebra **300A** such that their longitudinal centers are separate by a distance **314**. The distance **314** can vary depending upon the individual anatomy of the patient. According to certain embodiments, distance **314** is greater than distance **312**. For example, distance **314** can be about 10 mm to about 20 mm. Having distance **314** greater than distance **312** reduces destabilizing anterior-posterior and medial-lateral forces on vertebrae **300A** and **300B** created by the magnetic forces of the bone screws **100** and the bone screws 100'. Additionally, vertical distance **316** separating the bone screws **100** implanted in vertebral body **310A** of vertebra **300A** and the bone screws **100'** implanted in vertebral body **310B** of vertebra **300B** may be about 8 mm to about 12 mm.

According to these embodiments, and based upon computer modeling, the repulsive magnetic forces between the magnets (not shown in Figures 31A or 31B) of the bone screws **100** implanted in vertebral body **310A** of vertebra **300A** and the magnets (not shown in Figures 31A or 31B) of the bone screws **100'** implanted in vertebral body **310B** of vertebra **300B** may be about 2 N to about 10 N. According to some embodiments, the repulsive magnetic force is about 5 N. In this configuration, destabilizing anterior-posterior and medial-lateral forces on vertebrae **300A** and **300B** may be about 1 N or less.

In certain embodiments, and as shown in Figures 32A and 32B, disc-shaped housings **180** are inserted in each of vertebral bodies **310A** and **310B** of vertebrae **300A** and **300B,** respectively. According to these embodiments, a first disc-shaped housing **180** is implanted in vertebral body **310A** of vertebra **300A.** A second disc-shaped housing **180'** is implanted in vertebral body **310B** of adjacent vertebra **300B.** According to a preferred embodiment, the outer diameter **197** of disc-shaped housing **180** may be about 6 mm to about 15 mm. The outer diameter **197'** of disc-shaped housing **180'** is larger than diameter **197**. For example, outer diameter **197'** of disc-shaped housing **180'** may be about 8 mm to about 20 mm. Having diameter **197'** larger than diameter **197** reduces destabilizing anterior-posterior and medial-lateral forces on vertebrae **300A** and **300B** created by the magnetic forces of the disc-shaped housings. Additionally, vertical distance **318** separating the disc-shaped housing **180** implanted in vertebral body **310A** of vertebra **300A** and the disc-shaped housing **180'** implanted in vertebral body **310B** of vertebra **300B** may be about 8 mm to about 16 mm.

According to these embodiments, and based upon computer modeling, the repulsive magnetic forces between the magnet (not shown in Figures 32A or 32B) of the disc-shaped housing **180** implanted in vertebral body **310A** of vertebra **300A** and the magnet (not shown in Figures 32A or 32B) of the disc-shaped housing **180'** implanted in vertebral body **310B** of vertebra **300B** may be about 2 to about 10 N. According to certain embodiments the repulsive magnetic force is about 5 N. In this configuration, destabilizing anterior-posterior and medial-lateral forces on vertebrae **300A** and **300B** may be about 1 N or less.

### Use of the Bone Screws and Bone Plate on Intervertebral Joints

The bone screws and bone plate of the present invention may be used to stabilize and provide compression to an intervertebral joint undergoing fusion. In certain embodiments, the bone screws and bone plate of the present invention may be used to prevent or retard the deterioration of one or more nonfused intervertebral joints that are adjacent to the fused intervertebral joint.

"Fused intervertebral joint" refers to a joint between two vertebrae that is permanently connected, which eliminates the motion between the vertebrae. Fusion may occur by surgical methods known in the art, for example, through bone grafting-from the patient or donor, or with artificial bone substitutes-often with associated stabilizing implants such as plates and screws or rods and screws to help the vertebrae heal together. "Nonfused intervertebral joint" refers to a joint between two vertebrae that is not surgically connected or have not spontaneously fused.

"Deterioration" in this context refers to destruction of tissues within the intervertebral joint between two vertebrae including cartilage, annulus and disc nucleus materials. Deterioration may occur from the transfer of additional loads to a nonfused intervertebral joint that is above (i.e., superior) or below (i.e., inferior) the fused intervertebral joint. In particular, the loading may cause the adjacent nonfused intervertebral joint to compress, such that the vertebrae forming the joint are being pulled toward each other. Without being bound by theory, the present invention prevents and/or reduces deterioration by preventing or reducing the additional loading that is causing the nonfused intervertebral joint to compress.

Therefore, described herein is a method of stabilizing or providing compression at a fused intervertebral j oint or at an intervertebral joint undergoing fusion. Not falling within the scope of the invention, the method may comprise fastening a bone plate of the invention to the first vertebra and the second vertebra that form the fused/fusing intervertebral j oint, such that the bone plate bridges the intervertebral joint. The bone plate may be fastened by inserting a bone screw of the invention through each of the apertures of the bone plate and into the vertebra. The bone plate may be fastened to the vertebral bodies of the first and second vertebrae. The bone plate may be fastened such that the vertebra-facing surface of the bone plate is against the vertebral bodies.

A scope may be used to monitor the placement of the bone plate and the fastening of the screws. In some embodiments, intraoperative fluoroscopy is used.

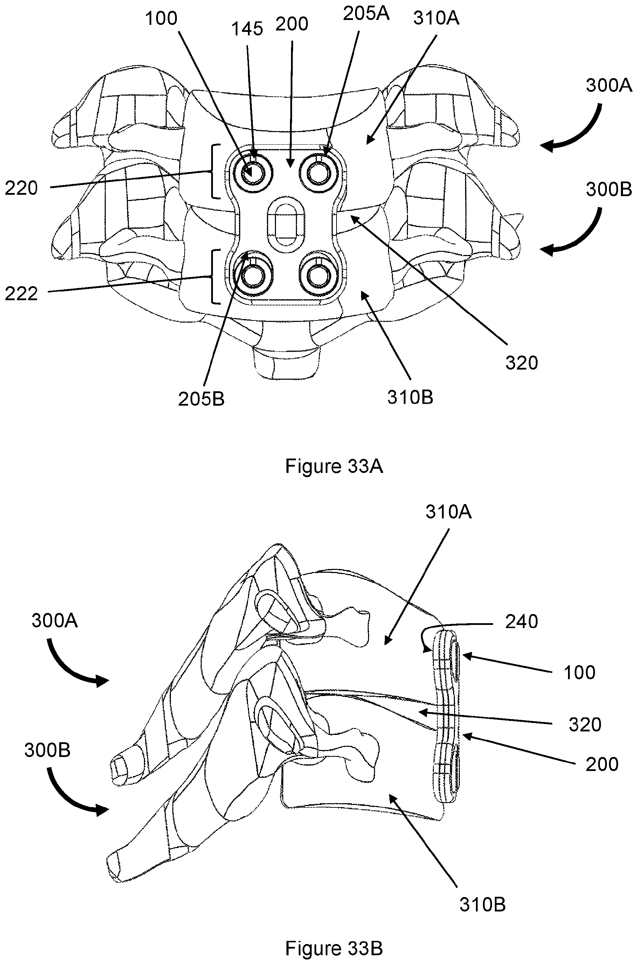

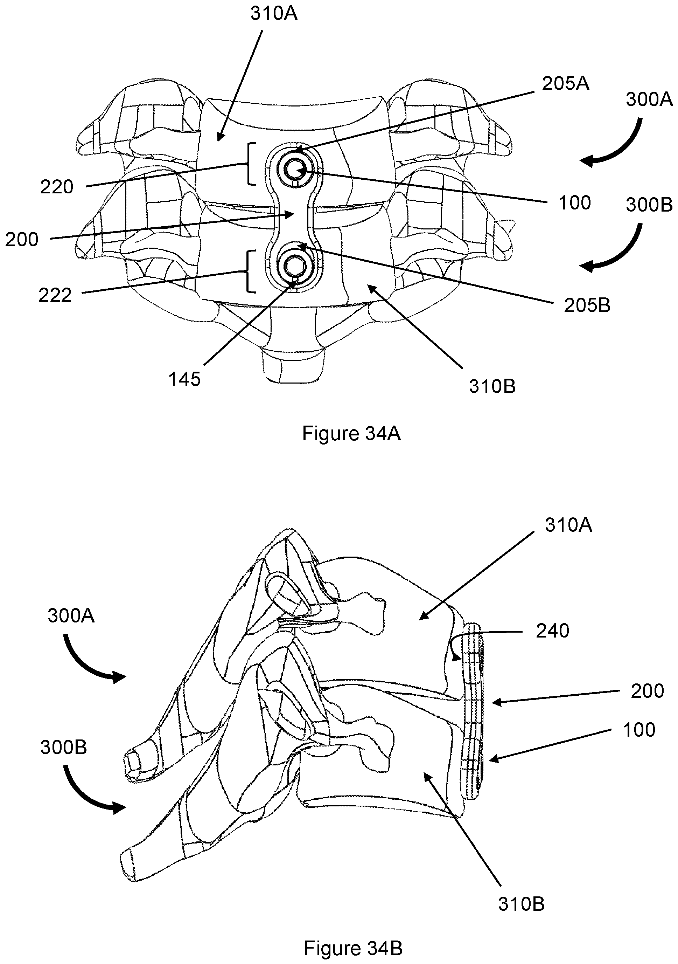

Figures 33A, 33B, 34A, and 34B illustrate the bone plate **200** fastened via one or more bone screws **100** to the vertebral body **310A** of a first superior vertebra **300A** and to the vertebral body **310B** of a second inferior vertebra **300B,** according to certain embodiments of the invention. The intervertebral joint **320** between the first vertebra **300A** and the second vertebra **300B** may be fused or undergoing fusion. Bone screw(s) **100** may be inserted into aperture(s) **205A** of the first end section **220** to fasten the bone plate **200** to the vertebral body **310A** of the first vertebra **300A**, and bone screw(s) **100** may be inserted into aperture(s) **205B** of the second end section **222** to fasten the bone plate **200** to the vertebral body **310B** of the second vertebra **300B.** The bone plate may be positioned such that the vertebra-facing surface **240** of the bone plate is against the vertebral body **310A** of the first vertebra **300A** and against the vertebral body **310B** of the first vertebra **300B.** In certain embodiments, bone screw(s) **100** may be inserted into aperture(s) **205A** of the first end section **220** to fasten the bone plate **200** to the vertebral body **310B** of the second vertebra **300B,** and bone screw(s) **100** may be inserted into aperture(s) **205B** of the second end section **222** to fasten the bone plate **200** to the vertebral body **310A** of the first vertebra **300A** (not shown).

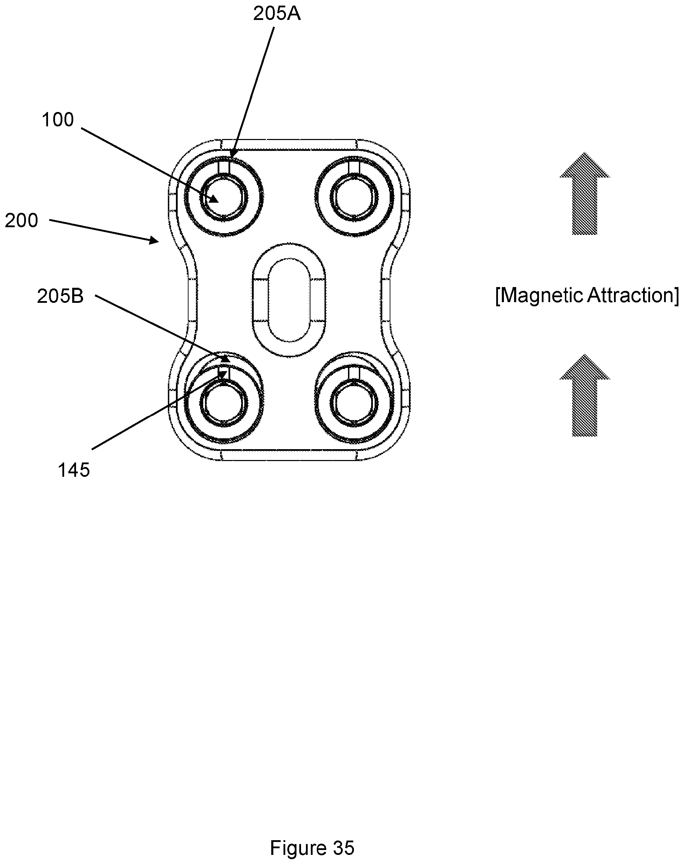

In certain embodiments, the bone screws used to fasten the bone plate to the vertebral body of the first vertebra and the vertebral body of the second vertebra may be oriented so that the polarity of the magnets within the bone screws generate a magnetic attraction between the magnet(s) of the bone screw(s) inserted into the vertebral body of the first vertebra and the magnet(s) of the bone screw(s) inserted into the vertebral body of the second vertebra. An example of such embodiments is illustrated in Figures 33A, 34A, and 35, in which the mark **145** on the bone screws **100** may be used to determine how to orient the screws **100** so that the bone screw(s) **100** in the aperture(s) **205A** and the bone screw(s) **100** in the aperture(s) **205B** are subject to an attractive force. These attractive forces between the magnet(s) (not shown in Figures 33A, 34A, or 35) of the bone screw(s) **100** in the aperture(s) **205A** and the magnet(s) (not shown in Figures 33A, 34A, or 35) of the bone screw(s) **100** in the aperture(s) **205B** may provide stability and/or provide compression at the fused/fusing intervertebral joint **320.** In some embodiments, due to the forces of magnetic attraction, the bone screw(s) **100** can move within the aperture(s) **205B**, which are shaped as elongated slots as shown in Figures 33A, 34A, and 35. Such movement can bring the bone screw(s) **100** in aperture(s) **205A** and the bone screw(s) **100** in the aperture(s) **205B** toward each other, thereby causing the first vertebra **300A** and the second vertebra **300B** to move towards each other. Such movement may also provide stability and/or provide compression at the fused/fusing intervertebral joint.

An aspect relates to a method of stabilizing fused intervertebral joints between three or more vertebrae (not shown). The method may comprise fastening a bone plate of the invention to each of the three or more vertebrae that form the fused/fusing intervertebral joints, such that the bone plate bridges the intervertebral joints. The bone plate may be fastened by inserting a bone screw of the invention through each of the apertures of the bone plate and into each of the vertebra. The bone plate may be fastened to the vertebral bodies of each vertebra. The bone plate may be fastened such that the vertebra-facing surface of the bone plate is against the vertebral bodies. For these methods, the bone plate may comprise a middle section that contains one or more rows of apertures. In certain embodiments, bone screws inserted through apertures of the first end section may be fastened to the superior-most vertebra, bone screws inserted through apertures of the second end section may be fastened to the inferior-most vertebra, and bone screws inserted through apertures of the middle section may be fastened to vertebrae therebetween. Alternatively, bone screws inserted through apertures of the first end section may be fastened to the inferior-most vertebra, bone screws inserted through apertures of the second end section may be fastened to the superior-most vertebra, and bone screws inserted through apertures of the middle section may be fastened to vertebrae therebetween.

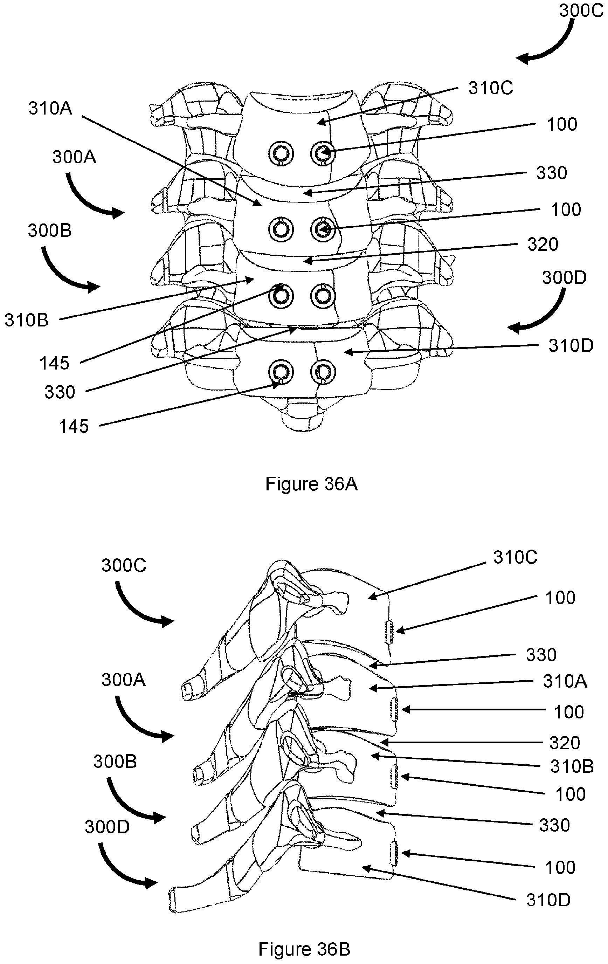

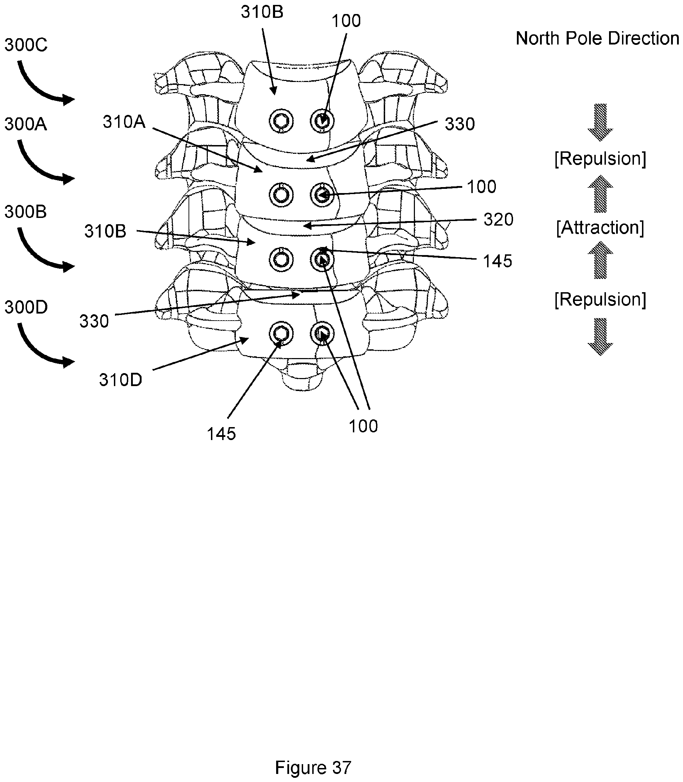

Another aspect is directed to a method of preventing or reducing deterioration of a nonfused intervertebral joint that is superior or inferior to a fused/fusing intervertebral joint. The method may comprise inserting one or more bone screws of the invention into a vertebral body of a first vertebra that forms part of the fused/fusing intervertebral joint, and inserting one or more bone screws of the invention into a vertebral body of an adjacent vertebra that does not form the fused/fusing intervertebral joint, such that the first vertebrae and the adjacent vertebrae form a nonfused intervertebral joint. The bone screws may be oriented so that their polarity generates a repulsive force between the magnet(s) of the bone screw(s) inserted into the first vertebra and the magnet(s) of the bone screw(s) inserted into the adjacent vertebra. Such a repulsive force prevents or reduces loading that may be caused by the presence of the fused intervertebral joint. An example of embodiments of the invention is illustrated in Figures 36A, 36B, and 37, in which first vertebra **300A** and second vertebra **300B** form a fused/fusing intervertebral joint **320;** vertebra **300C** forms a nonfused intervertebral joint **330** with vertebra **300A;** and vertebra **300D** forms a nonfused intervertebral j oint **330** with vertebra **300B**. Bone screws **100** are inserted into each of vertebral bodies **310A, 310B, 310C,** and **310D** of vertebrae **300A**, **300B**, **300C**, and **300D**, respectively. The marks **145** are used to orient the bone screws **100** such that the polarities of the magnets within the bone screws **100** generate an attractive force between the magnets (not shown in Figures 36A, 36B, or 37) of the bone screws fastened to vertebrae **300A** and **300B** (vertebrae between which there is a fused/fusing intervertebral joint **320**), a repulsive force between the magnets (not shown in Figures 36A, 36B, or 37) of the bone screws **100** fastened to vertebrae **300A** and **300C** (vertebrae between which there is a nonfused intervertebral joint **330**), and a repulsive force between the magnets (not shown in Figures 36A, 36B, or 37) of the bone screws **100** fastened to vertebrae **300B** and **300D** (vertebrae between which there is a nonfused intervertebral joint **330**) (see Figure 37). The repulsive forces can help prevent degeneration at the nonfused intervertebral joints.

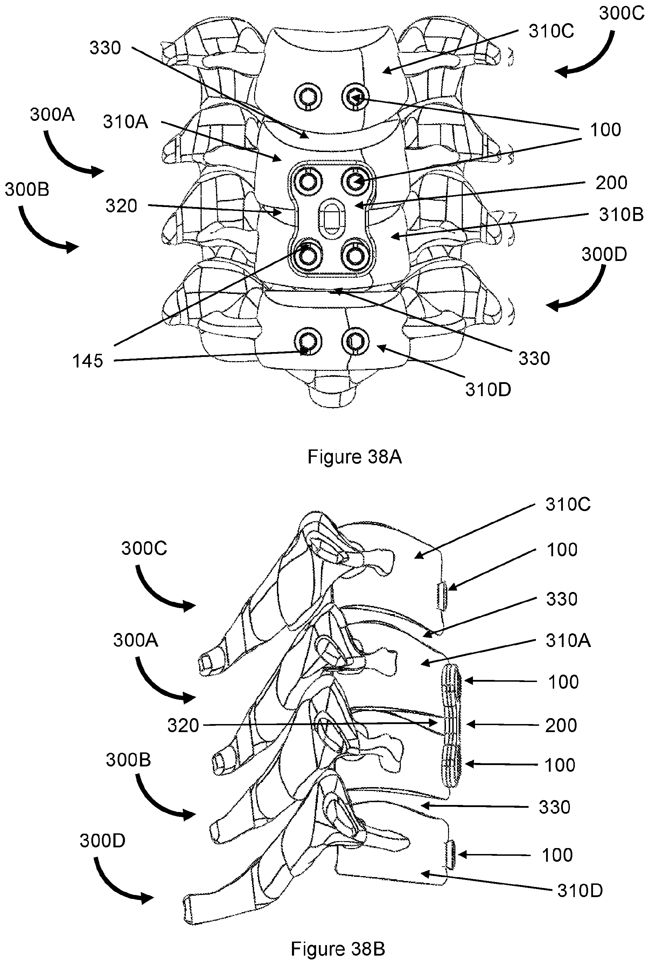

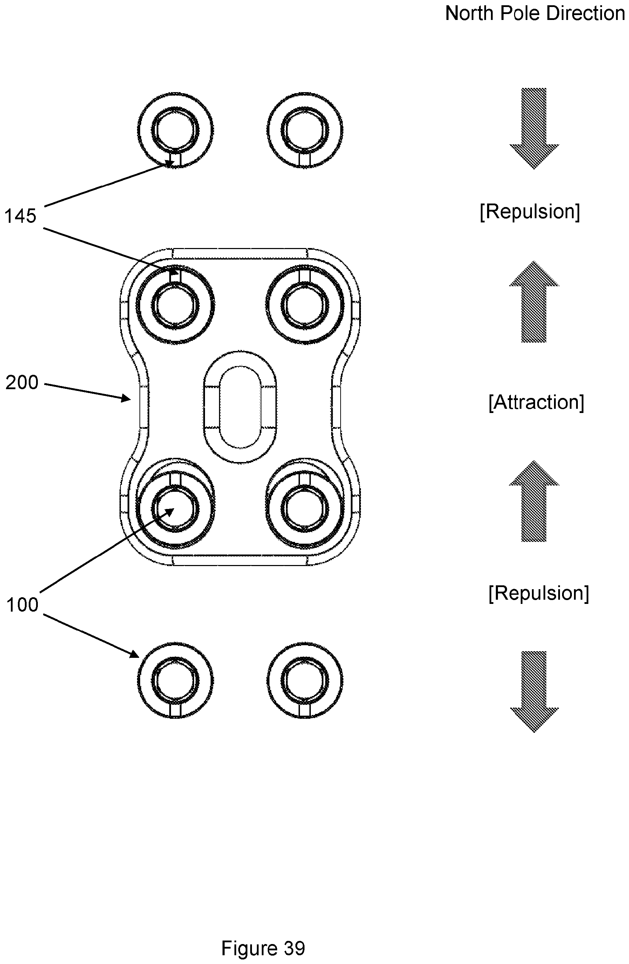

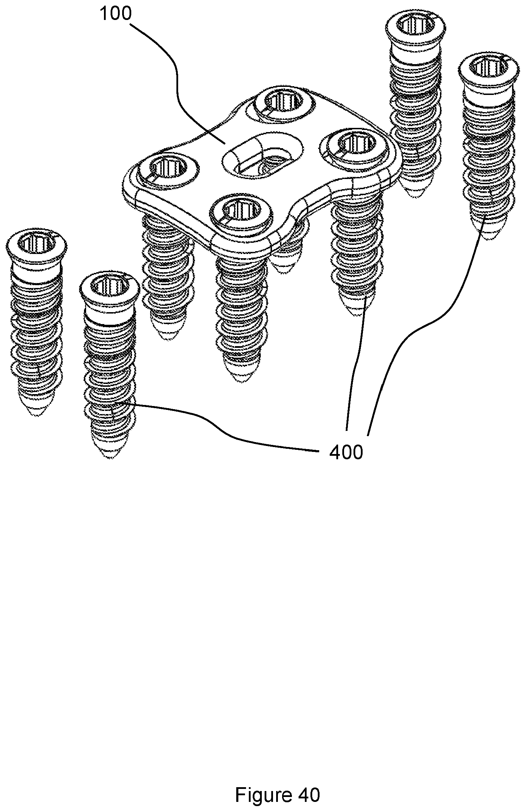

In some embodiments, bone screw(s) may be inserted into a vertebral body of one or more vertebrae superior or inferior to vertebrae of a fused/fusing intervertebral joint that are fastened with a bone plate. An example of such embodiments is illustrated in Figures 38A, 38B, and 40, which uses a bone plate **200** and bone screws **100** shown in Figure 39. A bone plate **200** may be fastened via bone screws **100** to a vertebral body **310A** of a vertebra **300A** and to a vertebral body **310B** of a vertebra **300B,** such that the bone plate **200** bridges a fused/fusing intervertebral joint **320** between vertebra **300A** and vertebra **300B.** Bone screws **100** may be inserted into a vertebral body **310C** of a third vertebra **300C** superior to the first vertebra **300A**, in which a nonfused intervertebral joint **330** is between the first vertebra **300A** and the third vertebra **300C**; and bone screws **100** may be inserted into a vertebral body **310D** of a fourth vertebra **300D** inferior to the second vertebra **300B,** in which a nonfused intervertebral joint **330** is between the second vertebra **300B** and the fourth vertebra **300D**. Guided by the marks **145**, the bone screws **100** may be oriented so that the polarity of the magnets within the bone screws **100** generates attractive and repulsive forces among magnets (not shown in Figures 38A, 38B, or 39) in the bone screws. As illustrated in Figure 39, the bone screws **100** may be oriented so that an attractive force is generated between the magnets (not shown in Figure 39) of the bone screws **100** inserted into the first vertebra **300A** and the magnets (not shown in Figure 39) of the bone screws **100** inserted into the second vertebra **300B.** In addition, a repulsive force is generated between the magnets (not shown in Figure 39) of the bone screws **100** inserted into the first vertebra **300A** and the magnets (not shown in Figure 39) of the bone screws **100** inserted into the third vertebra **300C**. Further, a repulsive force is generated between the magnets (not shown in Figure 39) of the bone screws **100** inserted into the second vertebra **300B** and the magnets (not shown in Figure 39) of the bone screws **100** inserted into the fourth vertebra **300D**.

An additional aspect of the invention relates to using the bone screws and/or bone plate of the invention to promote bone healing. In some embodiments, one or more bone screws may be inserted into each side of a bone fracture or break. The bone screws may be oriented to generate a magnetic attractive force between the magnet(s) of the one or more bone screws on one side of the fracture/break, and the magnet(s) of the one or more screws on the other side of the fracture/break.

In certain embodiments, the bone plate of the invention may be fastened to the fractured/broken bone, such that the plate bridges the fracture/break. Bone screws of the invention may be used to fasten the plate to the bone, and may be oriented to generate a magnetic attractive force between the magnet(s) of the bone screw(s) that fasten the bone plate to one side of the fracture/break, and the magnet(s) of the bone screw(s) that fasten the bone plate to the other side of the fracture/break.

The bone screws and/or bone plate of the invention may be used to promote healing in various types of bones, including long bones (e.g., humerus, radius, ulna, femur, tibia, fibula, etc.), short bones (e.g., metacarpals, phalanges, metatarsals, etc.), flat bones (e.g., scapula, ribs, sternum, etc.), and irregular bones (e.g., vertebrae, carpal bones, tarsal bones, etc.).

### Use of the Bone Screws and Disc-Shaped Housing on Intervertebral Joints

The bone screws and disc-shaped housings of the present invention may be used to exert a traction force on cervical vertebrae. The bone screws and disc-shaped housings of the present invention may also be used to treat pain caused by a herniated disk between a first vertebra and an adjacent vertebra and to treat a herniated disk between a first vertebra and an adjacent vertebra. In certain embodiments, the bone screws and disc-shaped housings of the present invention may be used to relax soft tissue and separate spine vertebral segments by imparting magnetic force(s) to separate and levitate cervical spine segments. The bone screws and disc-shaped housings are implanted into the superior and inferior vertebral bodies of the cervical spine motion segment where a traction force is desired. The traction force exerted by the bone screws and disc-shaped housings provides a consistent, continuous, low magnitude traction force, which maintain disc height and health and relieves acute and chronic cervical neck pain

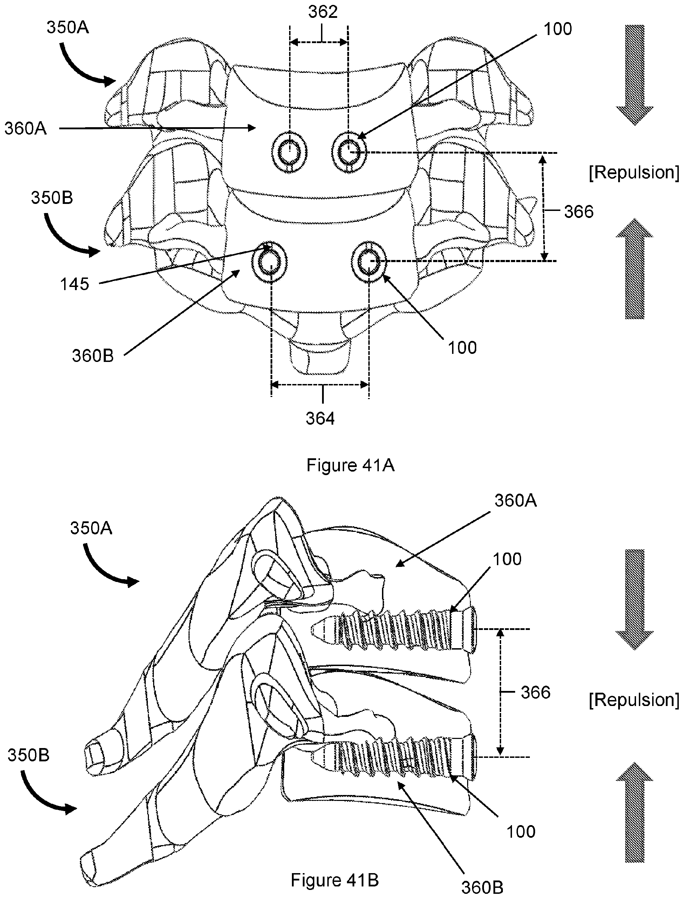

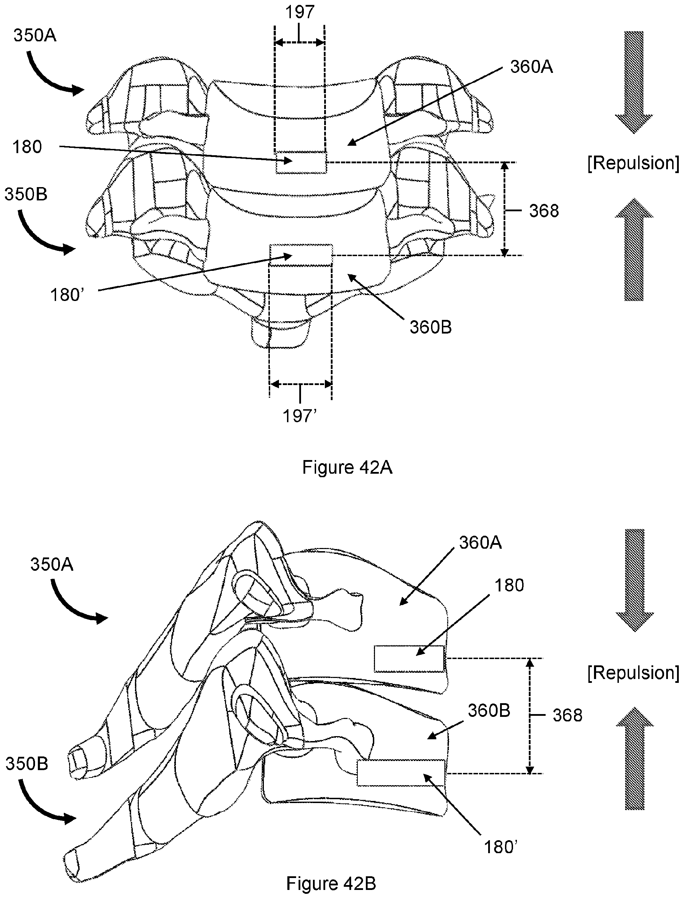

Figures 41A, 41B, 42A, and 42B illustrate a plurality of bone screws **100** and disc-shaped housings **180** implanted into the cervical vertebral body **360A** of a first superior vertebra **350A** and to the cervical vertebral body **360B** of a second, adjacent inferior vertebra **350B,** according to certain embodiments of the invention.

In certain embodiments of the invention, bone screws **100** are inserted into each of cervical vertebral bodies **360A** and **360B** of vertebrae **350A** and **350B,** respectively. The marks **145** are used to orient the bone screws **100** such that the polarities of the magnets within the bone screws **100** generate a repulsive force between the magnets (not shown in Figures 41A, 41B, 42A, or 42B) of the bone screws **100** fastened to vertebrae **350A** and **350B**.

According to embodiments of the invention, and as demonstrated in Figures 41A and 41B, two bone screws **100** are implanted into cervical vertebral body **360A** of vertebra **350A** such that their longitudinal centers are separate by a distance **362.** The distance **362** can vary depending upon the individual anatomy of the patient. For example, distance **362** can be about 8 to about 12 mm. Two bone screws **100** are also implanted into vertebral body **360B** of vertebra **350B** such that their longitudinal centers are separate by a distance **364**. The distance **364** can vary depending upon the individual anatomy of the patient. According to certain embodiments, distance **364** is greater than distance **362**. For example, distance **364** can be about 10 to about 20 mm. Having distance **364** greater than distance **362** reduces destabilizing anterior/posterior and medial/lateral forces on vertebrae **350A** and **350B** created by the magnetic forces of the bone screws **100.** Additionally, vertical distance **366** separating the bone screws **100** implanted into cervical vertebral body **360A** of vertebra **350A** and the bone screws **100** implanted into cervical vertebral body **360B** of vertebra **350B** may be about 8 to about 12 mm.

According to these embodiments, and based upon computer modeling, the repulsive magnetic forces between the magnets (not shown in Figures 41A or 41B) of the bone screws **100** implanted into cervical vertebral body **360A** of vertebra **350A** and the magnets (not shown in Figures 41A or 41B) of the bone screws **100** implanted into cervical vertebral body **360B** of vertebra **350B** may be about 2 to about 10 N. According to some embodiments, the repulsive magnetic force is about 5 N. In this configuration, destabilizing anterior/posterior and medial/lateral forces on vertebrae **350A** and **350B** may be about 1N or less.

In certain embodiments, and as shown in Figures 42A and 42B, disc-shaped housings **180** are inserted into each of cervical vertebral bodies **360A** and **360B** of vertebrae **350A** and **350B**, respectively. According to these embodiments, a first disc-shaped housing **180** is implanted into cervical vertebral body **360A** of vertebra **350A**. A second disc-shaped housing **180'** is also implanted into cervical vertebral body **360B** of adjacent vertebra **350B**. According to a preferred embodiment, the outer diameter **197** of disc-shaped housing **180** may be about 6 to about 15 mm. The outer diameter **197'** of disc-shaped housing **180'** is larger than diameter **197'. F**or example, outer diameter **197'** of disc-shaped housing **180'** may be about 8 to about 20 mm. Having diameter **197'** larger than diameter **197** reduces destabilizing anterior/posterior and medial/lateral forces on vertebrae **350A** and **350B** created by the magnetic forces of the disc-shaped housings. Additionally, vertical distance **368** separating the disc-shaped housing **180** implanted into cervical vertebral body **360A** of vertebra **360A** and the disc-shaped housing **180'** implanted into cervical vertebral body **360B** of vertebra **350B** may be about 8 to about 16 mm.

According to these embodiments, and based upon computer modeling, the repulsive magnetic forces between the magnet (not shown in Figures 42A or 42B) of the disc-shaped housing **180** implanted into cervical vertebral body **360A** of vertebra **350A** and the magnet (not shown in Figures 42A or 42B) of the disc-shaped housing **180'** implanted into cervical vertebral body **360B** of vertebra **350B** may be about 2 to about 10 N. According to certain embodiments the repulsive magnetic force is about 5 N. In this configuration, destabilizing anterior/posterior and medial/lateral forces on vertebrae **350A** and **350B** may be about 1 N or less.

### EXAMPLES

### Example 1

Modeling was used to study the impact on repulsion force of the size of the magnetic device, separation distance (distance between the magnetic device(s) in a first bone of the joint and the magnetic device(s) in a second bone of the joint), and the depth of the magnetic device (distance that the magnetic device extends into the bone, in some instances the length of the magnetic device). The magnetic forces were modeled using JMAG^{®}, a simulation technology that utilizes finite element analysis to calculate the magnetic forces and fields.

Table 1 below provides examples of repulsion forces generated from particular magnetic devices in certain arrangements. These examples show how changing the separation height can impact the repulsion force.

**Table 1. Repulsion forces generated from magnetic device(s) implanted in a first bone and a second bone of a joint, as determined using modeling.**

| **Magnetic Devices** | **Knee Flexion Angle** | **Magnet Depth** | **Separation Height** | **Repulsion Force** |
|---|---|---|---|---|
| A cylindrical magnetic device in the first bone and a disc-shaped magnetic device in the second bone (*see, e.g.,* Figures 13A and 13B) | 0° | 12.7 mm | 15 mm | 3.0 N |
| | | | | |
| Magnetic device of the first bone: | 0° | 12.7 mm | 20 mm | 1.1 N |
| • Diameter = 6.35 mm | | | | |
| • Length = 12.7 mm | | | | |
| | | | | |
| Magnetic device of the second bone: | | | | |
| • Diameter = 12.7 mm | | | | |
| • Height = 6.35 mm | | | | |
| Three identical cylindrical magnetic devices in the first bone and three identical cylindrical magnetic devices in the second bone (*see, e.g.,* Figures 7A and 7B) | 0° | 12.7 mm | 15 mm | 3.5 N |
| | 0° | 12.7 mm | 20 mm | 1.9 N |
| Magnetic devices of the first bone: | | | | |
| • Diameter = 4.76 mm | | | | |
| • Length = 12.7 mm | | | | |
| • Distance between each device = 2.38 mm | | | | |
| | | | | |
| Magnetic devices of the second bone: | | | | |
| • Diameter = 4.76 mm | | | | |
| • Length = 12.7 mm | | | | |
| • Distance between each device = 2.38 mm | | | | |
| Three identical cylindrical magnetic devices in the first bone and two identical cylindrical magnetic devices in the second bone (*see, e.g.,* Figures 14A-14C) | 0° | 12.7 mm | 15 mm | 1.59 N |
| Magnetic devices of the first bone: | 45° | 12.7 mm | 15 mm | 1.0 N |
| • Diameter = 4.76 mm | | | | |
| • Length = 12.7 mm | | | | |
| • Distance between each device = 7.94 mm | | | | |
| | 90° | 12.7 mm | 15 mm | 1.59 N |
| Magnetic devices of the second bone: | | | | |
| • Diameter = 4.76 mm | | | | |
| • Length = 12.7 mm | | | | |
| • Distance between each device = 7.94 mm | | | | |
| A rectangular prism magnetic device in the first bone and a rectangular prism magnetic device in the second bone (see, e.g., Figures 11A and 11B) | 0° | 12.7 mm | 15 mm | 13.0 N |
| | | | | |
| Magnetic device of the first bone: | | | | |
| • Length = 12.7 mm | | | | |
| • Width = 19.05 mm | | | | |
| • Height = 6.35 mm | 0° | 12.7 mm | 20 mm | 5.8 N |
| | | | | |
| Magnetic device of the second bone: | | | | |
| • Length = 12.7 mm | | | | |
| • Width = 19.05 mm | | | | |
| • Height = 6.35 mm | | | | |
| A plate with a casing that encases a magnetic device in the first bone, and a plate with a casing that encases a magnetic device in the second bone *(see,* e.g., Figures 18A and 18B) | | | | |
| | | | | |
| Magnetic device of the first bone: | 0° | 12.7 mm | 2.5 mm | 41.8 N |
| • Length of plate= 12.7 mm | | | | |
| • Width of plate = 19.05 mm | | | | |
| • Height of plate = 6.35 mm | | | | |
| • Length of protrusion = 12.7 mm | | | | |
| • Width of protrusion = 19.05 mm | | | | |
| • Height of protrusion = 6.35 mm | | | | |
| | | | | |
| Magnetic device of the second bone: | 0° | 12.7 mm | 15 mm | 13.0 N |
| • Length of plate= 12.7 mm | | | | |
| • Width of plate = 19.05 mm | | | | |
| • Height of plate = 6.35 mm | | | | |
| • Length of protrusion = 12.7 mm | | | | |
| • Width of protrusion = 19.05 mm | | | | |
| • Height of protrusion = 6.35 mm | | | | |

### Example 2

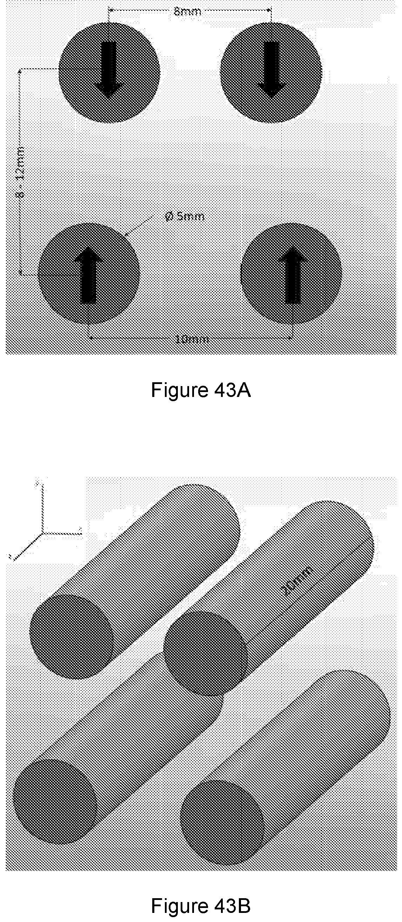

A series of finite element analyses were conducted using JMAG to study the magnetic forces that are generated when magnets are used in a cervical traction application. In these studies, four magnets are placed in a configuration depicted in Figures 43A and 43B. These magnets are 5 mm in diameter and 20 mm in length. The lower two magnets are 10 mm apart and the upper two magnets are 8 mm apart. The height difference between the upper and lower magnets is varied at 8, 10, and 12 mm.

The analyses determined the repulsion force that was created by the magnets in this configuration. The axial loads generated when the height difference between the upper and lower magnets is 8 mm, 10 mm, and 12 mm are provided in Tables 2, 3, and 4, respectively. These distances are the minimum axial distances for the example given; therefore, the axial loads that are generated represent the maximum axial forces. Values could be changed depending on the patient, for example, the anatomical dimensions of the patient's vertebrae.

**Table 2. Axial loads generated when the height difference between the upper and lower magnets is 8 mm.**

| **Magnet** | **X-Force (N)** | **Y-Force (N)** | **Z-Force (N)** |
|---|---|---|---|
| Upper Left | -4.8499 | 5.7862 | 0.0051 |
| Upper Right | 4.8245 | 5.7942 | -0.0275 |
| Lower right | 6.4334 | -5.7086 | -0.0429 |
| Lower left | -6.5100 | -5.8269 | -0.0238 |

**Table 3. Axial loads generated when the height difference between the upper and lower magnets is 10 mm.**

| **Magnet** | **X-Force (N)** | **Y-Force (N)** | **Z-Force (N)** |
|---|---|---|---|
| Upper Left | -6.2114 | 3.0589 | -0.0518 |
| Upper Right | 6.2109 | 2.9520 | 0.0510 |
| Lower right | 4.9500 | -3.0811 | -0.0345 |
| Lower left | -4.9628 | -3.0309 | -0.0041 |

**Table 4. Axial loads generated when the height difference between the upper and lower magnets is 12 mm.**

| **Magnet** | **X-Force (N)** | **Y-Force (N)** | **Z-Force (N)** |
|---|---|---|---|
| Upper Left | -6.5264 | 2.1109 | -0.0304 |
| Upper Right | 6.5149 | 1.8994 | 0.0233 |
| Lower right | 4.2294 | -1.8929 | -0.0362 |
| Lower left | -4.2380 | -1.9811 | 0.0011 |

The foregoing description is given for clearness of understanding only, and no unnecessary limitations should be understood therefrom, as modifications within the scope of the invention may be apparent to those having ordinary skill in the art.

Detailed embodiments of the present magnetic devices are disclosed herein: however, it is to be understood that the disclosed embodiments are merely illustrative and that the magnetic devices may be embodied in various forms. In addition, each of the examples given in connection with the various embodiments of the systems are intended to be illustrative, and not restrictive.

Throughout this specification and the claims which follow, unless the context requires otherwise, the word "comprise" and variations such as "comprises" and "comprising" will be understood to imply the inclusion of a stated integer or step or group of integers or steps but not the exclusion of any other integer or step or group of integers or steps.

Throughout the specification, where compositions are described as including components or materials, it is contemplated that the compositions can also consist essentially of, or consist of, any combination of the recited components or materials, unless described otherwise. Likewise, where methods are described as including particular steps, it is contemplated that the methods can also consist essentially of, or consist of, any combination of the recited steps, unless described otherwise.

## Claims

1. A bone screw (100) comprising
(a) a shaft (103) comprising:
(i) a head (105), a lower section (115), and a middle section therebetween (110), wherein the shaft (103) comprises a circular cross-section;
(ii) an outer wall surface (120), wherein a plurality of threads (122) is disposed along at least a portion of the outer wall surface (120); and
(iii) a bore (130) defining an inner wall surface (125) and located in the middle section (110), wherein the bore (130) comprises a substantially cylindrical shape; and
(b) a magnet (135) configured to fit within the bore (130), wherein the magnet (135) is a rare-earth magnet,
**characterized in that** the bore (130) comprises a substantially cylindrical shape containing a flattened side (137) in the direction of the long axis of the bore (130); and wherein the magnet (135) comprises a substantially cylindrical shape containing a flattened side (137) in the direction of the long axis of the magnet (135).

2. The bone screw (100) of claim 1, wherein the magnet (135) is adhered to at least a portion of the inner wall surface (125).

3. The bone screw (100) of any one of claims 1-2, wherein the lower section (115) of the shaft (103) is attached to the middle section (110).

4. The bone screw (100) of any one of claims 1-3, wherein the lower section (115) is hermetically sealed to the middle section (110).

5. The bone screw (100) of any one of claims 1-4, wherein the head (105) of the shaft (103) comprises an end surface (107) that is configured to receive a driver.

6. The bone screw (100) of any one of claims 1-5, wherein the end surface (107) of the head (105) contains a mark (145) that identifies the polarity of the magnet (135).

7. The bone screw 100 of any one of claims 1-6 for use in reducing loading across a cartilaginous joint, wherein the cartilaginous joint is a knee joint or an intervertebral joint.

8. An apparatus comprising:
(1) plate (200) comprising a first end section (220), a second end section (222), and a middle section (221) therebetween; and a first surface (240) and a second surface (241); wherein
(a) the first end section (220) comprises one or more apertures (205A), wherein the one or more apertures (205A) of the first end section (220) is substantially circular;
(b) the second end section (222) comprises one or more apertures (205B), wherein the one or more apertures (205B) of the second end section (222) is substantially an elongated slot; and
(2) at least two bone screws (100) of any one of claims 1-7.

9. The apparatus of claim 8, wherein the first surface (240) of the plate (200) comprises a contouring profile configured to the contouring profile of vertebral bodies of adjacent vertebrae.

10. The apparatus of claim 8 or 9, wherein the first surface (240) comprises a contouring profile configured to the contouring profile of vertebral bodies of adjacent vertebrae.

11. The apparatus of any one of claims 8-10, wherein two apertures (205A) are in the first end section (220) and two apertures (205B) are in the second end section (222).

12. The apparatus of any one of claims 8-11 for use in stabilizing a fused intervertebral joint, or for use in preventing or reducing deterioration of a nonfused intervertebral joint.

## Patentansprüche

1. Knochenschraube (100), umfassend
(a) einen Schaft (103), der umfasst:
(i) einen Kopf (105), einen unteren Abschnitt (115) und einen dazwischen liegenden mittleren Abschnitt (110), wobei der Schaft (103) einen kreisförmigen Querschnitt aufweist;
(ii) eine Außenwandfläche (120), wobei eine Vielzahl von Gewinden (122) entlang mindestens eines Teils der Außenwandfläche (120) angeordnet ist; und
(iii) eine Bohrung '(130), die eine Innenwandfläche (125) definiert und in dem mittleren Abschnitt (110) angeordnet ist, wobei die Bohrung (130) eine im Wesentlichen zylindrische Form aufweist; und
(b) einen Magneten (135), der so gestaltet ist, dass er in die Bohrung (130) passt, wobei der Magnet (135) ein Seltenerdmagnet ist,
**dadurch gekennzeichnet, dass** die Bohrung (130) eine im Wesentlichen zylindrische Form aufweist, die eine abgeflachte Seite (137) in der Richtung der langen Achse der Bohrung (130) enthält; und wobei der Magnet (135) eine im Wesentlichen zylindrische Form aufweist, die eine abgeflachte Seite (137) in der Richtung der langen Achse des Magneten (135) enthält.

2. Knochenschraube (100) nach Anspruch 1, wobei der Magnet (135) an mindestens einen Teil der Innenwandfläche (125) geklebt ist.

3. Knochenschraube (100) nach einem der Ansprüche 1 bis 2, wobei der untere Abschnitt (115) des Schafts (103) am mittleren Abschnitt (110) befestigt ist.

4. Knochenschraube (100) nach einem der Ansprüche 1 bis 3, wobei der untere Abschnitt (115) zum mittleren Abschnitt (110) hermetisch abgedichtet ist.

5. Knochenschraube (100) nach einem der Ansprüche 1 bis 4, wobei der Kopf (105) des Schafts (103) eine Endfläche (107) aufweist, die zur Aufnahme eines Antriebs ausgebildet ist.

6. Knochenschraube (100) nach einem der Ansprüche 1 bis 5, wobei die Endfläche (107) des Kopfes (105) eine Markierung (145) enthält, die die Polarität des Magneten (135) kennzeichnet.

7. Knochenschraube (100) nach einem der Ansprüche 1 bis 6 zur Verwendung bei der Verringerung der Belastung in einem Knorpelgelenk, wobei das Knorpelgelenk ein Kniegelenk oder ein Zwischenwirbelgelenk ist.

8. Vorrichtung, die Folgendes umfasst:
(1) eine Platte (200), die einen ersten Endabschnitt (220), einen zweiten Endabschnitt (222) und einen dazwischen liegenden mittleren Abschnitt (221) sowie eine erste Oberfläche (240) und eine zweite Oberfläche (241) aufweist, wobei
(a) der erste Endabschnitt (220) eine oder mehrere Öffnungen (205A) aufweist, wobei die eine oder mehreren Öffnungen (205A) des ersten Endabschnitts (220) im Wesentlichen kreisförmig ist/sind;
(b) der zweite Endabschnitt (222) eine oder mehrere Öffnungen (205B) aufweist, wobei die eine oder mehreren Öffnungen (205B) des zweiten Endabschnitts (222) im Wesentlichen ein länglicher Schlitz ist/sind; und
(2) mindestens zwei Knochenschrauben (100) nach einem der Ansprüche 1 bis 7.

9. Vorrichtung nach Anspruch 8, wobei die erste Oberfläche (240) der Platte (200) ein Konturprofil aufweist, das an das Konturprofil von Wirbelkörpern benachbarter Wirbel angepasst ist.

10. Vorrichtung nach Anspruch 8 oder 9, wobei die erste Oberfläche (240) ein Konturprofil aufweist, das an das Konturprofil von Wirbelkörpern benachbarter Wirbel angepasst ist.

11. Vorrichtung nach einem der Ansprüche 8-10, wobei sich zwei Öffnungen (205A) im ersten Endabschnitt (220) und zwei Öffnungen (205B) im zweiten Endabschnitt (222) befinden.

12. Vorrichtung nach einem der Ansprüche 8-11 zur Verwendung bei der Stabilisierung eines verschmolzenen Zwischenwirbelgelenks oder zur Verwendung bei der Verhinderung oder Verringerung der Verschlechterung eines nicht verschmolzenen Zwischenwirbelgelenks.

## Revendications

1. Vis à os (100) comprenant :
(a) un arbre (103) comprenant :
(i) une tête (105), une section inférieure (115), et une section centrale entre elles (110), où l'arbre (103) comprend une section transversale circulaire ;
(ii) une surface de paroi externe (120), dans laquelle une pluralité de filets (122) est disposée le long d'une partie au moins de la surface de paroi externe (120) ; et
(iii) un alésage (130) définissant une surface de paroi interne (125) et situé dans la section centrale (110), où l'alésage (130) comprend une forme sensiblement cylindrique ; et
(b) un aimant (135) configuré pour s'adapter dans l'alésage (130), où l'aimant (135) est un aimant de terres rares,
**caractérisée en ce que** l'alésage (130) comprend une forme sensiblement cylindrique contenant un côté aplati (137) dans la direction de l'axe long de l'alésage (130) ; et où l'aimant (135) comprend une forme sensiblement cylindrique contenant un côté aplati (137) dans la direction de l'axe long de l'aimant (135).

2. Vis à os (100) selon la revendication 1, dans laquelle l'aimant (135) est adhéré à au moins une partie de la surface de paroi interne (125).

3. Vis à os (100) selon l'une quelconque des revendications 1-2, dans laquelle la section inférieure (115) de l'arbre (103) est attachée à la section centrale (110).

4. Vis à os (100) selon l'une quelconque des revendications 1-3, dans laquelle la partie inférieure (115) est hermétiquement scellée à la partie centrale (110).

5. Vis à os (100) selon l'une quelconque des revendications 1-4, dans laquelle la tête (105) de l'arbre (103) comprend une surface d'extrémité (107) qui est configurée pour recevoir un tournevis.

6. Vis à os (100) selon l'une quelconque des revendications 1-5, dans laquelle la surface d'extrémité (107) de la tête (105) contient une marque (145) qui identifie la polarité de l'aimant (135).

7. Vis à os 100 selon l'une quelconque des revendications 1-6, pour utilisation dans la réduction d'une charge au travers d'une articulation cartilagineuse, où l'articulation cartilagineuse est une articulation de genou ou une articulation intervertébrale.

8. Appareil comprenant :
(1) une plaque (200) comprenant une première section d'extrémité (220), une deuxième section d'extrémité (222) et une section centrale (221) entre elles ; et une première surface (240) et une deuxième surface (241) ; dans laquelle
(a) la première section d'extrémité (220) comprend une ou plusieurs ouvertures (205A), dans laquelle l'une ou les plusieurs ouvertures (205A) de la première section d'extrémité (220) sont sensiblement circulaires ;
(b) la deuxième section d'extrémité (222) comprend une ou plusieurs ouvertures (205B), dans laquelle l'une ou les plusieurs ouvertures (205B) de la deuxième section d'extrémité (222) sont sensiblement des fentes allongées ; et
(2) au moins deux vis à os (100) selon l'une quelconque des revendications 1-7.

9. Appareil selon la revendication 8, dans lequel la première surface (240) de la plaque (200) comprend un profil de contour configuré au profil de contour de corps vertébraux de vertèbres adjacentes.

10. Appareil selon la revendication 8 ou 9, dans lequel la première surface (240) comprend un profil de contour configuré au profil de contour de corps vertébraux de vertèbres adjacentes.

11. Appareil selon l'une quelconque des revendications 8-10, dans lequel deux ouvertures (205A) sont dans la première section d'extrémité (220) et deux ouvertures (205B) sont dans la deuxième section d'extrémité (222).

12. Appareil selon l'une quelconque des revendications 8-11, pour utilisation dans la stabilisation d'une articulation intervertébrale fusionnée, ou pour utilisation dans la prévention ou la réduction de détérioration dans une articulation intervertébrale non fusionnée.
